# EUROPEAN PATENT SPECIFICATION

(11) **EP 1 673 354 B1**
(45) Date of publication and mention of the grant of the patent: **22.04.2009**
(21) Application number: 03759708.5
(22) Date of filing: 03.10.2003
(51) Int. Cl.: C07D 239/70, C07D 405/04, C07D 401/04, C07D 417/04, C07D 409/04, C07D 401/12, C07D 405/12, C07D 401/06, C07D 221/16, A61K 31/505, A61K 31/5355

(54) **ARYLINDENOPYRIDINES AND ARYLINDENOPYRIMIDINES AND THEIR USE AS ADENOSINE A2A RECEPTOR ANTAGONISTS**
ARYLINDENOPYRIDIN- UND ARYLINDENOPYRIMIDINVERBINDUNGEN UND IHRE VERWENDUNG ALS ADENOSIN-A2A-REZEPTOR ANTAGONISTEN
ARYLINDENOPYRIDINES ET ARYLINDENOPYRIMIDINES ET LEUR UTILISATION EN TANT QU'ANTAGONISTE DES RECEPTEURS D'ADENOSINE A2A

(43) Date of publication of application: 28.06.2006
(73) Proprietor: Ortho-McNeil Pharmaceutical, Inc., Raritan, NJ 08869-0606 (US)
(72) Inventor: HEINTZELMAN, Geoffrey, R., Annandale, NJ 08801 (US); BULLINGTON, James, L., Hamilton Square, NJ 08690-1945 (US); RUPERT, Kenneth, C., South Orange, NJ 07079 (US)
(74) Representative: Mercer, Christopher Paul
(86) International application number: PCT/US2003/031471
(87) International publication number: WO 2005/042500

(56) References cited:
- WO-A-01/62233
- WO-A-93/08167
- WO-A-02/085894
- WO-A-03/088963
- JP-A- 2001 139 556
- EL-TAWEEL F M A ET AL: "SYNTHETIC ROUTES TO FLUORENONE, INDENOPYIRDINE, 4H-NAPHTHOÄ2,1-BÜPYRANS AND PYRIDINE DERIVATIVES" BOLLETTINO CHIMICO FARMACEUTICO, SOCIETA EDITORIALE FARMACEUTICA, MILANO, IT, vol. 140, no. 5, 2001, pages 306-310, XP009025181 ISSN: 0006-6648
- GOERLITZER K ET AL: "INDENOÄ1,2-DÜPYRIMIDIN-4-YL-AMINE INDENOÄ1,2ÜPYRIMIDIN-4-YL-AMINES" PHARMAZIE, VEB VERLAG VOLK UND GESUNDHEIT. BERLIN, DD, vol. 52, no. 9, 1997, pages 670-672, XP001179310 ISSN: 0031-7144
- BURGER K ET AL: "TRIFLUORMETHYL-SUBSTITUIERTE PYRIMIDINE AUS ENAMINEN UND TRIFLUORACETONITRIL TRIFLUOROMETHYL-SUBSTITUTED PYRIMIDINES FROM ENAMINES AND TRIFLUOROACETONITRILE" LIEBIGS ANNALEN DER CHEMIE, VERLAG CHEMIE GMBH. WEINHEIM, DE, vol. 5, 1984, pages 991-1002, XP001179309 ISSN: 0170-2041
- DEMERAC S ET AL: "5H-INDENOÄ1,2-DÜPYRIMIDIN-5-ONES" AUSTRALIAN JOURNAL OF CHEMISTRY, XX, XX, vol. 25, 1972, pages 2651-2657, XP009025111 ISSN: 0004-9425
- AUGUSTIN M: "SYNTHESE UND REAKTIONEN VON 2-ÄBIS-(ALKYLTHIO)-METHYLIDENÜ-INDAN-1,3- DIONEN SYNTHESIS AND REACTIONS OF 2-ÄBIS-(ALKYLTHIO)-METHYLIDENÜ-INDAN-1,3-D ION" JOURNAL FUER PRAKTISCHE CHEMIE, WILEY, WEINHEIM, DE, vol. 321, no. 2, 1979, pages 205-214, XP009025109 ISSN: 1436-9966
- N EL-RAYYES: "Heterocycles. 14. Synthesis of 5H-Indenopyrimidines" J.CHEM.ENG.DATA, vol. 32, 1987, pages 481-483, XP002270517
- KAPPE C O ET AL: "SYNTHESIS AND REACTIONS OF BIGINELLI-COMPOUNDS. PART I" JOURNAL OF HETEROCYCLIC CHEMISTRY, HETEROCORPORATION. PROVO, US, January 1989 (1989-01), pages 55-64, XP002952094 ISSN: 0022-152X
- A ROSOWSKY: "One step synthesis of novel 2,4-diaminopyrimidine antifolates" J HETEROCYCLCI CHEMISTRY, vol. 36, 1999, pages 723-728, XP002270518
- KANDEEL E M ET AL: "SYNTHESIS OF NEW 1,2-DIHYDRO-4-AMINO-2-THIOXO-5H-INDENO 1,2-D PYRIMIDIN-5-ONE DERIVATIVES" PAKISTAN JOURNAL OF SCIENTIFIC AND INDUSTRIAL RESEARCH, XX, XX, vol. 29, no. 6, December 1986 (1986-12), pages 424-426, XP009025178 ISSN: 0030-9885

## Description

### Field of the Invention

This invention relates to novel arylindenopyridines and arylindenopyrimidines, particularly for therapeutic and prophylactic uses. Disorders that may be treated and/or prevented using these compounds include neurodegenerative and movement disorders ameliorated by antagonizing Adenosine A2a receptors.

### Background of the Invention

### Adenosine A2a Receptors

Adenosine is a purine nucleotide produced by all metabolically active cells within the body. Adenosine exerts its effects via four subtypes of cell-surface receptors (A1, A2a, A2b and A3), which belong to the G protein coupled receptor superfamily (Stiles, G.L. Journal of Biological Chemistry, 1992, 267, 6451). A1 and A3 couple to inhibitory G protein, while A2a and A2b couple to stimulatory G protein. A2a receptors are mainly found in the brain, both in neurons and glial cells (highest level in the striatum and nucleus accumbens, moderate to high level in olfactory tubercle, hypothalamus, and hippocampus etc. regions) (Rosin, D. L.; Robeva, A.; Woodard, R. L.; Guyenet, P. G.; Linden, J. Journal of Comparative Neurology ,1998, 401, 163).

In peripheral tissues, A2a receptors are found in platelets, neutrophils, vascular smooth muscle and endothelium (Gessi, S.; Varani, K.; Merighi, S.; Ongini, E.; Borea, P. A. British Journal of Pharmacology, 2000, 129, 2). The striatum is the main brain region for the regulation of motor activity, particularly through its innervation from dopaminergic neurons originating in the substantia nigra. The striatum is the major target of the dopaminergic neuron degeneration in patients with Parkinson's Disease (PD). Within the striatum, A2a receptors are co-localized with dopamine D2 receptors, suggesting an important site for the integration of adenosine and dopamine signaling in the brain (Fink, J. S.; Weaver, D. R.; Rivkees, S. A.; Peterfreund, R. A.; Pollack, A. E.; Adler, E. M.; Reppert, S. M. Brain Research Molecular Brain Research, 1992, 14, 186).

Neurochemical studies have shown that activation of A2a receptors reduces the binding affinity of D2 agonist to their receptors. This D2R and A2aR receptor-receptor interaction has been demonstrated in striatal membrane preparations of rats (Ferre, S.; von Euler, G.; Johansson, B.; Fredholm, B. B.; Fuxe, K. Proceedings of the National Academy of Sciences of the United States of America, 1991, 88, 7238) as well as in fibroblast cell lines after transfected with A2aR and D2R cDNAs (Salim, H.; Ferre, S.; Dalal, A.; Peterfreund, R. A.; Fuxe, K.; Vincent, J. D.; Lledo, P. M. Journal of Neurochemistry, 2000, 74, 432). In vivo, pharmacological blockade of A2a receptors using A2a antagonist leads to beneficial effects in dopaminergic neurotoxin MPTP(1-methyl-4-pheny-1,2,3,6-tetrahydropyridine)-induced PD in various species, including mice, rats, and monkeys (Ikeda, K.; Kurokawa, M.; Aoyama, S.; Kuwana, Y. Journal of Neurochemistry, 2002, 80, 262). Furthermore, A2a knockout mice with genetic blockade of A2a function have been found to be less sensitive to motor impairment and neurochemical changes when they were exposed to neurotoxin MPTP (Chen, J. F.; Xu, K.; Petzer, J. P.; Staal, R.; Xu, Y. H.; Beilstein, M.; Sonsalla, P. K.; Castagnoli, K.; Castagnoli, N., Jr.; Schwarzschild, M. A. Journal of Neuroscience, 2001, 21, RC143).

In humans, the adenosine receptor antagonist theophylline has been found to produce beneficial effects in PD patients (Mally, J.; Stone, T. W. Journal of the Neurological Sciences, 1995, 132, 129). Consistently, recent epidemiological study has shown that high caffeine consumption makes people less likely to develop PD (Ascherio, A.; Zhang, S. M.; Hernan, M. A.; Kawachi, I.; Colditz, G. A.; Speizer, F. E.; Willett, W. C. Annals of Neurology, 2001, 50, 56). In summary, adenosine A2a receptor blockers may provide a new class of antiparkinsonian agents (Impagnatiello, F.; Bastia, E.; Ongini, E.; Monopoli, A. Emerging Therapeutic Targets, 2000, 4, 635).

EI-Taweel et al (2001) Bollettino Chimico Farmaceutico, 140(5): 306-310 discloses synthetic routes to fluorenone, indenopyirdine, 4h-naphtho[2,1-b]pyrans and pyridine derivatives.

Goerlitzer et al (1997) Veb Verlag Volk Und Gesundheit, 52(9): 670-672 discloses the synthesis of indeno[1,2-d]pyrimidin-4-yl-amines.

Burger et al (1984) Liebigs Annalen Der Chemie, 5:991-1002 discloses the synthesis of trifluoromethyl-substituted pyrimidines from enamines and trifluoroacetonitrile.

Demerac et al (1972) Australian Journal Of Chemistry, 25:2651-2657 discloses the synthesis of 5H-indeno[1,2-d]pyrimidin-5-ones.

Augustin (1979) Journal Fuer Praktische Chemie, 321(2):205-214 discloses the synthesis and reactions of 2-[Bis-(alkylthio)-methyliden]-indan-1,3-dionen.

EI-Rayyes (1987) J. Chem. Eng. Data, 32:481-483 discloses the synthesis of 5H Indenopyrimidines.

Kappe et al (1989) Journal Of Heterocyclic Chemistry, 1:55-64 discloses the synthesis and reactions of "Biginelli" compounds (2-oxo(or thioxo)-1,2,3,4-tetrahydropyrimidine-5-carboxylic acid derivatives.

JP2001139556 discloses the production of indenopyridine and indenopyrimidine compounds useful in medicine and methods for producing the same.

WO 93/08167 discloses compounds represented by general formula (A), a salt thereof, a process for the production thereof, and an agrohorticultural bactericide containing same.

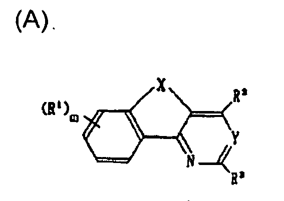

Rosowsky J Heterocyclci Chemistry, 36:723-728 discloses the one step synthesis of 2,4 diaminopyrimidine antifolates.

Kandeel et al (1986) Pakistan Journal Of Scientific And Industrial Research, 29(6):424 426 discloses the synthesis of 1,2-dihydro-4-amino-2-thioxo-5h-indeno 1,2-d pyrimidin-5-one derivatives.

WO 02/085894 discloses arylindenopyridines, and pharmaceutical compositions comprising same, said to be useful for treating disorders ameliorated by reducing PDE activity in appropriate cells. Therapeutic and prophylactic methods using the pharmaceutical compositions are disclosed.

WO 03/088963 discloses arylindenopyridines, and pharmaceutical compositions comprising same, said to be useful for treating disorders ameliorated by antagonizing Adensine A2a receptors or reducing PDE activity in appropriate cells. Therapeutic and prophylactic methods using the pharmaceutical compositions are disclosed.

WO 01/62233 discloses cyclic heteroaromatic compounds, containing at least one nitrogen atom, and to their use in the manufacture of medicaments for the treatment of diseases, related to adenosine receptor modulators, such as Alzheimer's disease, Parkinson's disease, neuroprotection, schizophrenia, anxiety, pain, respiration deficits, depression, asthma, allergic responses, hypoxia, ischaemia, seizure, substance abuse, sedation and they may be active as muscle relaxants, antipsychotics, antiepileptics, anticonvulsants and cardioprotective agents.

### Summary of the Invention

This invention provides a compound having the structure of Formula I or II 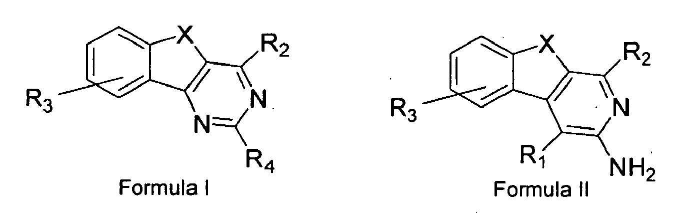 or a pharmaceutically acceptable salt thereof, wherein in Formula I:
(b) R₂ is selected from the group consisting of optionally substituted aryl, and optionally substituted heteroaryl;
(c) R₃ is from one to four groups independently selected from the group consisting of:
   hydrogen, halo, C₁₋₈ straight or branched chain alkyl, arylalkyl, C₃₋₇ cycloalkyl, C₁₋₈ alkoxy, cyano, C₁₋₄ carboalkoxy, trifluoromethyl, C₁₋₈ alkylsulfonyl, halogen, nitro, hydroxy, trifluoromethoxy, C₁₋₈ carboxylate, aryl, heteroaryl, and heterocyclyl, -NR₁₁R₁₂,
   wherein R₁₁ and R₁₂ are independently selected from H, C₁₋₈ straight or branched chain alkyl, arylalkyl, C₃₋₇ cycloalkyl, carboxyalkyl, aryl, heteroaryl, and heterocyclyl or R₁₀ and R₁₁ taken together with the nitrogen form a heteroaryl or heterocyclyl group,
   -NR₁₃COR₁₄,
   wherein R₁₃ is selected from hydrogen or alkyl and R₁₄ is selected from hydrogen, alkyl, substituted alkyl, C₁₋₃ alkoxyl, carboxyalkyl, aryl, arylalkyl, heteroaryl, heterocyclyl, R₁₅R₁₆N (CH₂)ₚ-, or R₁₅R₁₆NCO(CH₂)ₚ-,
   wherein R₁₅ and R₁₆ are independently selected from H, OH, alkyl, and alkoxy, and p is an integer from 1-6, wherein the alkyl group may be substituted with carboxyl, alkyl, aryl, substituted aryl, heterocyclyl, substituted heterocyclyl, heteroaryl, substituted heteroaryl, hydroxamic acid, sulfonamide, sulfonyl, hydroxy, thiol, alkoxy or arylalkyl, or R₁₃ and R₁₄ taken together with the carbonyl form a carbonyl containing heterocyclyl group;
(d) R₄ is selected from the group consisting of hydrogen, C₁₋₆ straight or branched chain alkyl, benzyl
   wherein the alkyl and benzyl groups are optionally substituted with one or more groups selected from C₃₋₇ cycloalkyl, C₁₋₈ alkoxy, cyano, C₁₋₄ carboalkoxy, trifluoromethyl, C₁₋₈ alkylsulfonyl, halogen, nitro, hydroxy, trifluoromethoxy, C₁₋₈ carboxylate, amino, NR₁₇R₁₈, aryl and heteroaryl,
   -OR₁₇, and -NR₁₇R₁₈,
   wherein R₁₇ and R₁₈ are independently selected from hydrogen, and optionally substituted C₁₋₆ alkyl or aryl; and
(e) X is selected from C=S, C=O; CH₂, CHOH, CHOR₁₉; or CHNR₂₀R₂₁ where R₁₉, R₂₀, and R₂₁ are selected from optionally substituted C₁₋₈ straight of branched chain alkyl, wherein the substituents on the alkyl group are selected from C₁₋₈ alkoxy, hydroxy, halogen, amino, cyano, or NR₂₂R₂₃ wherein R₂₂ and R₂₃ are independently selected from the group consisting of hydrogen, C₁₋₈ straight or branched chain alkyl, C₃₋₇ cycloalkyl, benzyl, aryl, heteroaryl, or NR₂₂R₂₃ taken together from a heterocycle or heteroaryl;
with the proviso that said compound is not: 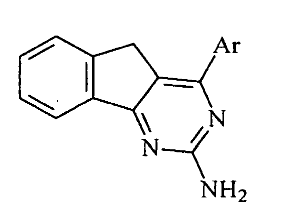 wherein Ar is phenyl, *p*-methoxyphenyl, *m*-methoxyphenyl,
*p*-bromophenyl, *m*-bromophenyl, *p*-chlorophenyl, *o*-chlorophenyl, 1-naphthyl, 2-naphthyl, 2-thienyl or 2-furanyl; or 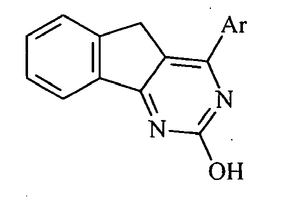 wherein Ar is *p*-chlorophenyl, 1-naphthyl, or 2-thienyl;
and in Formula II:
(a) R₁ is selected from the group consisting of
   (i) -COR₅, wherein R₅ is selected from H, optionally substituted C₁₋₈ straight or branched chain alkyl, optionally substituted aryl and optionally substituted arylalkyl;
      wherein the substituents on the alkyl, aryl and arylalkyl group are selected from C₁₋₈ alkoxy, phenylacetyloxy, hydroxy, halogen, p-tosyloxy, mesyloxy, amino, cyano, carboalkoxy, or NR₇R₈ wherein R₇ and R₈ are independently selected from the group consisting of hydrogen, C₁₋₈ straight or branched chain alkyl, C₃₋₇ cycloalkyl, benzyl, aryl, or heteroaryl or NR₇R₈ taken together form a heterocycle or heteroaryl;
   (ii) COOR₅, wherein R₅ is as defined above;
   (iii) cyano;
   (iv) -CONR₉R₁₀ wherein R₉ and R₁₀ are independently selected from H, C₁₋₈ straight or branched chain alkyl, C₃₋₇ cycloalkyl, trifluoromethyl, hydroxy, alkoxy, acyl, alkylcarbonyl, carboxyl, arylalkyl, aryl, heteroaryl and heterocyclyl;
      wherein the alkyl, cycloalkyl, alkoxy, acyl, alkylcarbonyl, carboxyl, arylalkyl, aryl, heteroaryl and heterocyclyl groups may be substituted with carboxyl, alkyl, aryl, substituted aryl, heterocyclyl, substituted heterocyclyl, heteroaryl, substituted heteroaryl, hydroxamic acid, sulfonamide, sulfonyl, hydroxy, thiol, amino, alkoxy or arylalkyl, or R₉ and R₁₀ taken together with the nitrogen to which they are attached form a heterocycle or heteroaryl group;
   (v) optionally substituted C₁₋₈ straight or branched chain alkyl;
      wherein the substituents on the alkyl, group are selected from C₁₋₈ alkoxy, phenylacetyloxy, hydroxy, halogen, p-tosyloxy, mesyloxy, amino, cyano, carboalkoxy, carboxyl, aryl, heterocyclyl, heteroaryl, sulfonyl, thiol, alkylthio, or NR₇R₈ wherein R₇ and R₈ are as defined above;
(b) R₂ is selected from the group consisting of optionally substituted alkyl, optionally substituted aryl, optionally substituted heteroaryl, optionally substituted heterocyclyl and optionally substituted C₃₋₇ cycloalkyl, C₁₋₈ alkoxy, aryloxy, C₁₋₈ alkylsulfonyl, arylsulfonyl, arylthio, C₁₋₈ alkylthio, or -NR₂₄R₂₅
   wherein R₂₄ and R₂₅ are independently selected from H, C₁₋₈ straight or branched chain alkyl, arylalkyl, C₃₋₇ cycloalkyl, carboxyalkyl, aryl, heteroaryl, and heterocyclyl or R₂₄ and R₂₅ taken together with the nitrogen form a heteroaryl or heterocyclyl group,
(c) R₃ is from one to four groups independently selected from the group consisting of:
   hydrogen, halo, C₁₋₈ straight or branched chain alkyl, arylalkyl, C₃₋₇ cycloalkyl, C₁₋₈ alkoxy, cyano, C₁₋₄ carboalkoxy, trifluoromethyl, C₁₋₈ alkylsulfonyl, halogen, nitro, hydroxy, trifluoromethoxy, C₁₋₈ carboxylate, aryl, heteroaryl, and
   heterocyclyl, -NR₁₁R₁₂,
   wherein R₁₁ and R₁₂ are independently selected from H, C₁₋₈ straight or branched chain alkyl, arylalkyl, C₃₋₇ cycloalkyl, carboxyalkyl, aryl, heteroaryl, and heterocyclyl or R₁₀ and R₁₁ taken together with the nitrogen form a heteroaryl or heterocyclyl group,
   -NR₁₃COR₁₄,
   wherein R₁₃ is selected from hydrogen or alkyl and R₁₄ is selected from hydrogen, alkyl, substituted alkyl, C₁₋₃ alkoxyl, carboxyalkyl, aryl, arylalkyl, heteroaryl, heterocyclyl, R₁₅R₁₆N (CH₂)ₚ-, or R₁₅R₁₆NCO(CH₂)ₚ-, wherein R₁₅ and R₁₆ are independently selected from H, OH, alkyl, and alkoxy, and p is an integer from 1-6, wherein the alkyl group may be substituted with carboxyl, alkyl, aryl, substituted aryl, heterocyclyl, substituted heterocyclyl, heteroaryl, substituted heteroaryl,
   hydroxamic acid, sulfonamide, sulfonyl, hydroxy, thiol, alkoxy or arylalkyl, or R₁₃ and R₁₄ taken together with the carbonyl form a carbonyl containing heterocyclyl group;
(e) X is selected from C=S, C=O; CH₂, CHOH, CHOR₁₉; or CHNR₂₀R₂₁ where R₁₉, R₂₀, and R₂₁ are selected from optionally substituted C₁₋₈ straight of branched chain alkyl, wherein the substituents on the alkyl group are selected from C₁₋₈ alkoxy, hydroxy, halogen, amino, cyano, or NR₂₂R₂₃ wherein R₂₂ and R₂₃ are independently selected from the group consisting of hydrogen, C₁₋₈ straight or branched chain alkyl, C₃₋₇ cycloalkyl, benzyl, aryl, heteroaryl, or NR₂₂R₂₃ taken together from a heterocycle or heteroaryl;
with the proviso that when R₁ is a cyano, then R₂ is not phenyl.

This invention also provides a pharmaceutical composition comprising the instant compound and a pharmaceutically acceptable carrier. In this embodiment, the proviso relating to the compounds of Formula I does not apply.

This invention further provides the instant compound or pharmaceutical composition for treating a subject having a condition ameliorated by antagonizing Adenosine A2a receptors. In this embodiment, the proviso relating to the compounds of Formula I does not apply.

This invention further provides the instant compound or pharmaceutical composition for preventing a disorder ameliorated by antagonizing Adenosine A2a receptors in a subject. In this embodiment, the proviso relating to the compounds of Formula I does not apply.

### Detailed Description of the Invention

Compounds of Formula I are potent small molecule antagonists of the Adenosine A2a receptors that have demonstrated potency for the antagonism of Adenosine A2a, A1, and A3 receptors.

Preferred embodiments for R₁ are COOR₅ wherein R₅ is an optionally substituted C₁₋₈ straight or branched chain alkyl. Preferably the alkyl chain is substituted with a dialkylamino group.

Preferred embodiments for R₂ in Formula II are optionally substituted heteroaryl and optionally substituted aryl. Preferably, in Formula I and Formula II R₂ is an optionally substituted furan.

Preferred substituents for R₃ include hydrogen, halo, hydroxy, amino, trifluoromethyl, alkoxy, hydroxyalkyl chains, and aminoalkyl chains,

Preferred substituents for R₄ include NH₂ and alkylamino.

In a preferred embodiment, the compound is selected from the group of compounds shown in Tables 1 and 2 hereinafter.

More preferably, the compound is selected from the following compounds:

The compound of claim 1, formula I, wherein R₄ is amino. 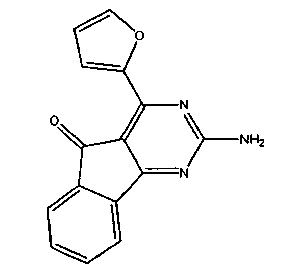 2-amino-4-furan-2-yl-indeno[1,2-d]pyrimidin-5-one 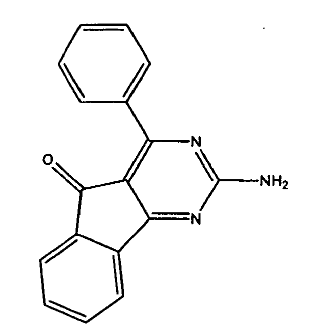 2-amino-4-phenyl-indeno[1,2-d]pyrimidin-5-one 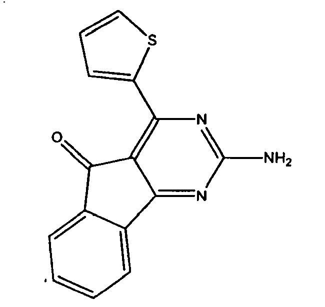 2-amino-4-thiophen-2-yl-indeno[1,2-d]pyrimidin-5-one 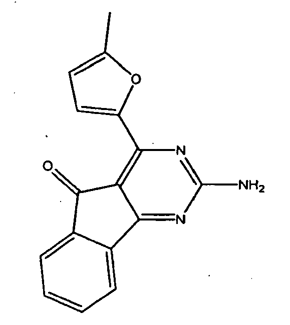 2-amino-4-(5-methyl-furan-2-yl)-indeno[1,2-d]pyrimidin-5-one 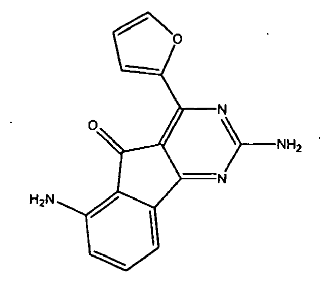 2,6-diamino-4-furan-2-yl-indeno[1,2-d]pyrimidin-5-one 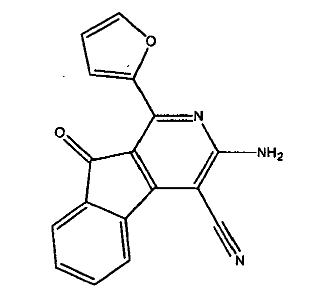 9*H*-indeno[2,1-c]pyridine-4-carbonitrile, 3-amino-1-furan-2-yl-9-oxo- 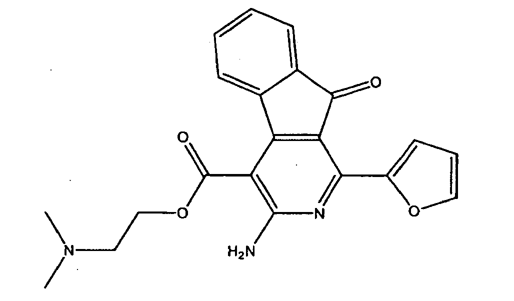 9*H*-indeno[2,1-c]pyridine-4-carboxylic acid, 3-amino-1-furan-2-yl-9-oxo-, 2-dimethylamino-ethyl ester 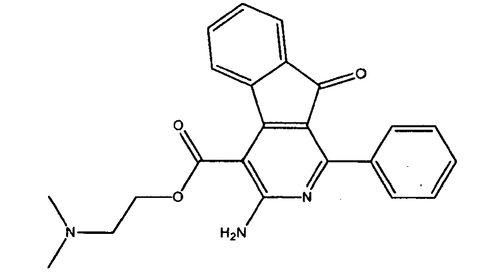 9*H*-indeno[2,1-c]pyridine-4-carboxylic acid, 3-amino-1-phenyl-9-oxo-, 2-dimethylamino-ethyl ester 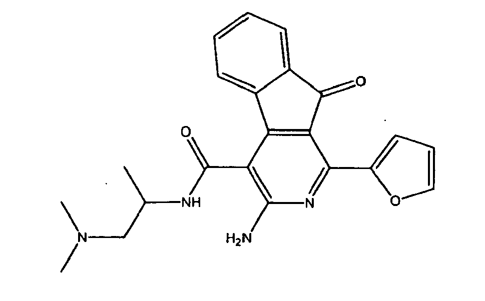 9*H*-indeno[2,1-c]pyridine-4-carboxylic acid, 3-aminol-furan-2-yl-9-oxo-, (2-dimethylamino-1-methyl-ethyl)-amide 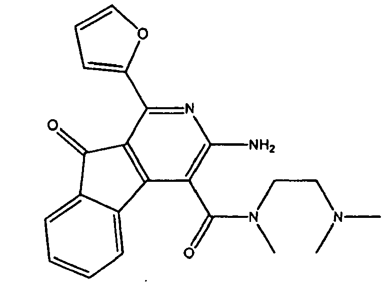 9*H*-indeno[2,1-c]pyridine-4-carboxylic acid, 3-amino-1-furan-2-yl-9-oxo-, (2-dimethylamino-ethyl)-methyl-amide . 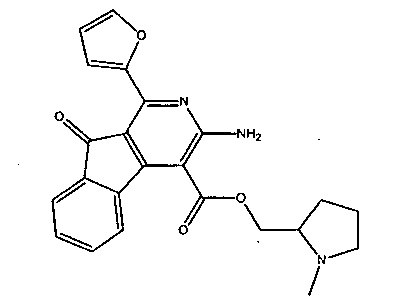 9*H*-indeno[2,1-c]pyridine-4-carboxylic acid, 3-amino-1-furan-2-yl-9-oxo-, 1-methyl-pyrrolidin-2-ylmethyl ester

The instant compounds can be isolated and used as free bases. They can also be isolated and used as pharmaceutically acceptable salts. Examples of such salts include hydrobromic, hydroiodic, hydrochloric, perchloric, sulfuric, maleic, fumaric, malic, tartaric, citric, benzoic, mandelic, methanesulfonic, hydroethanesulfonic, benzenesulfonic, oxalic, palmoic, 2-naphthalenesulfonic, p-toluenesulfonic, cyclohexanesulfamic and saccharic.

This invention also provides a pharmaceutical composition comprising the instant compound and a pharmaceutically acceptable carrier. In this embodiment, the proviso relating to the compounds of Formula I does not apply.

Pharmaceutically acceptable carriers are well known to those skilled in the art and include, but are not limited to, from about 0.01 to about 0.1 M and preferably 0.05 M phosphate buffer or 0.8% saline. Such pharmaceutically acceptable carriers can be aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, ethanol, alcoholic/aqueous solutions, glycerol, emulsions or suspensions, including saline and buffered media. Oral carriers can be elixirs, syrups, capsules, tablets and the like. The typical solid carrier is an inert substance such as lactose, starch, glucose, methyl-cellulose, magnesium stearate, dicalcium phosphate, mannitol and the like. Parenteral carriers include sodium chloride solution, Ringer's dextrose, dextrose and sodium chloride, lactated Ringer's and fixed oils. Intravenous carriers include fluid and nutrient replenishers, electrolyte replenishers such as those based on Ringer's dextrose and the like. Preservatives and other additives can also be present, such as, for example, antimicrobials, antioxidants, chelating agents, inert gases and the like. All carriers can be mixed as needed with disintegrants, diluents, granulating agents, lubricants, binders and the like using conventional techniques known in the art.

This invention further provides the instant compounds and compositions for treating a subject having a condition ameliorated by antagonizing Adenosine A2a receptors. In this embodiment, the proviso relating to the compounds of Formula I does not apply.

In one embodiment, the disorder is a neurodegenerative or movement disorder. Examples of disorders treatable by the instant pharmaceutical composition include, without limitation, Parkinson's Disease, Huntington's Disease, Multiple System Atrophy, Corticobasal Degeneration, Alzheimer's Disease, and Senile Dementia.

In one preferred embodiment, the disorder is Parkinson's disease.

As used herein, the term "subject" includes, without limitation, any animal or artificially modified animal having a disorder ameliorated by antagonizing adenosine A2a receptors. In a preferred embodiment, the subject is a human.

Administering the instant pharmaceutical composition can be effected or performed using any of the various methods known to those skilled in the art. The instant compounds can be administered, for example, intravenously, intramuscularly, orally and subcutaneously. In the preferred embodiment, the instant pharmaceutical composition is administered orally. Additionally, administration can comprise giving the subject a plurality of dosages over a suitable period of time. Such administration regimens can be determined according to routine methods.

As used herein, a "therapeutically effective dose" of a pharmaceutical composition is an amount sufficient to stop, reverse or reduce the progression of a disorder. A "prophylactically effective dose" of a pharmaceutical composition is an amount sufficient to prevent a disorder, i.e., eliminate, ameliorate and/or delay the disorder's onset. Methods are known in the art for determining therapeutically and prophylactically effective doses for the instant pharmaceutical composition. The effective dose for administering the pharmaceutical composition to a human, for example, can be determined mathematically from the results of animal studies.

In one embodiment, the therapeutically and/or prophylactically effective dose is a dose sufficient to deliver from about 0.001 mg/kg of body weight to about 200 mg/kg of body weight of the instant pharmaceutical composition. In another embodiment, the therapeutically and/or prophylactically effective dose is a dose sufficient to deliver from about 0.05 mg/kg of body weight to about 50 mg/kg of body weight. More specifically, in one embodiment, oral doses range from about 0.05 mg/kg to about 100 mg/kg daily. In another embodiment, oral doses range from about 0.05 mg/kg to about 50 mg/kg daily, and in a further embodiment, from about 0.05 mg/kg to about 20 mg/kg daily. In yet another embodiment, infusion doses range from about 1.0 µg/kg/min to about 10 mg/kg/min of inhibitor, admixed with a pharmaceutical carrier over a period ranging from about several minutes to about several days. In a further embodiment, for topical administration, the instant compound can be combined with a pharmaceutical carrier at a drug/carrier ratio of from about 0.001 to about 0.1.

### Definitions and Nomenclature

Unless otherwise noted, under standard nomenclature used throughout this disclosure the terminal portion of the designated side chain is described first, followed by the adjacent functionality toward the point of attachment.

As used herein, the following chemical terms shall have the meanings as set forth in the following paragraphs: "independently", when in reference to chemical substituents, shall mean that when more than one substituent exists, the substituents may be the same or different;.

"Alkyl" shall mean straight, cyclic and branched-chain alkyl. Unless otherwise stated, the alkyl group will contain 1-20 carbon atoms. Unless otherwise stated, the alkyl group may be optionally substituted with one or more groups such as halogen, OH, CN, mercapto, nitro, amino, C₁-C₈-alkyl, C₁-C₈-alkoxyl, C₁-C₈-alkylthio, C₁-C₈-alkyl-amino, di(C₁-C₈-alkyl)amino, (mono-, di-, tri-, and per-) halo-alkyl, formyl, carboxy, alkoxycarbonyl, C₁-C₈-alkyl-CO-O-, C₁-C₈-alkyl-CO-NH-, carboxamide, hydroxamic acid, sulfonamide, sulfonyl, thiol, aryl, aryl(c₁-c₈)alkyl, heterocyclyl, and heteroaryl.

"Alkoxy" shall mean -O-alkyl and unless otherwise stated, it will have 1-8 carbon atoms.

The term "bioisostere" is defined as "groups or molecules which have chemical and physical properties producing broadly similar biological properties." (Burger's Medicinal Chemistry and Drug Discovery, M. E. Wolff, ed. Fifth Edition, Vol. 1, 1995, Pg. 785).

"Halogen" shall mean fluorine, chlorine, bromine or iodine; "PH" or "Ph" shall mean phenyl; "Ac" shall mean acyl; "Bn" shall mean benzyl.

The term "acyl" as used herein, whether used alone or as part of a substituent group, means an organic radical having 2 to 6 carbon atoms (branched or straight chain) derived from an organic acid by removal of the hydroxyl group. The term "Ac" as used herein, whether used alone or as part of a substituent group, means acetyl.

"Aryl" or "Ar," whether used alone or as part of a substituent group, is a carbocyclic aromatic radical including, but not limited to, phenyl, 1- or 2-naphthyl and the like. The carbocyclic aromatic radical may be substituted by independent replacement of 1 to 5 of the hydrogen atoms thereon with halogen, OH, CN, mercapto, nitro, amino, C₁-C₈-alkyl, C₁-C₈-alkoxyl, C₁-C₈-alkylthio, C₁-C₈-alkyl-amino, di(C₁-C₈-alkyl)amino, (mono-, di-, tri-, and per-) halo-alkyl, formyl, carboxy, alkoxycarbonyl, C₁-C₈-alkyl-CO-O-, C₁-C₈-alkyl-CO-NH-, or carboxamide. Illustrative aryl radicals include, for example, phenyl, naphthyl, biphenyl, fluorophenyl, difluorophenyl, benzyl, benzoyloxyphenyl, carboethoxyphenyl, acetylphenyl, ethoxyphenyl, phenoxyphenyl, hydroxyphenyl, carboxyphenyl, trifluoromethylphenyl, methoxyethylphenyl, acetamidophenyl, tolyl, xylyl, dimethylcarbamylphenyl and the like. "Ph" or "PH" denotes phenyl.

Whether used alone or as part of a substituent group, "heteroaryl" refers to a cyclic, fully unsaturated radical having from five to ten ring atoms of which one ring atom is selected from S, O, and N; 0-2 ring atoms are additional heteroatoms independently selected from S, O, and N; and the remaining ring atoms are carbon. The radical may be joined to the rest of the molecule via any of the ring atoms. Exemplary heteroaryl groups include, for example, pyridinyl, pyrazinyl, pyrimidinyl, pyridazinyl, pyrroyl, pyrazolyl, imidazolyl, thiazolyl, oxazolyl, isoxazolyl, thiadiazolyl, triazolyl, triazinyl, oxadiazolyl, thienyl, furanyl, quinolinyl, isoquinolinyl, indolyl, isothiazolyl, 2-oxazepinyl, azepinyl, N-oxo-pyridyl, 1-dioxothienyl, benzothiazolyl, benzoxazolyl, benzothienyl, quinolinyl-N-oxide, benzimidazolyl, benzopyranyl, benzisothiazolyl, benzisoxazolyl, benzodiazinyl, benzofurazanyl, benzothiopyranyl, indazolyl, indolizinyl, benzofuryl, chromonyl, coumarinyl, cinnolinyl, quinoxalinyl, indazolyl, pyrrolopyridinyl, furopyridinyl (such as furo[2,3-c]pyridinyl, furo[3,2-b]pyridinyl, or furo[2,3-b]pyridinyl), imidazopyridinyl (such as imidazo[4,5-b]pyridinyl or imidazo[4,5-c]pyridinyl), naphthyridinyl, phthalazinyl, purinyl, pyridopyridyl, quinazolinyl, thienofuryl, thienopyridyl, thienothienyl, and furyl. The heteroaryl group may be substituted by independent replacement of 1 to 5 of the hydrogen atoms thereon with halogen, OH, CN, mercapto, nitro, amino, C₁-C₈-alkyl, C₁-C₈-alkoxyl, C₁-C₈-alkylthio, C₁-C₈-alkyl-amino, di(C₁-C₈-alkyl)amino, (mono-, di-, tri-, and per-) halo-alkyl, formyl, carboxy, alkoxycarbonyl, C₁-C₈-alkyl-CO-O-, C₁-C₈-alkyl-CO-NH-, or carboxamide. Heteroaryl may be substituted with a mono-oxo to give for example a 4-oxo-1H-quinoline.

The terms "heterocycle," "heterocyclic," and "heterocyclo" refer to an optionally substituted, fully or partially saturated cyclic group which is, for example, a 4- to 7-membered monocyclic, 7- to 11-membered bicyclic, or 10-to 15-membered tricyclic ring system, which has at least one heteroatom in at least one carbon atom containing ring. Each ring of the heterocyclic group containing a heteroatom may have 1, 2, or 3 heteroatoms selected from nitrogen atoms, oxygen atoms, and sulfur atoms, where the nitrogen and sulfur heteroatoms may also optionally be oxidized. The nitrogen atoms may optionally be quaternized. The heterocyclic group may be attached at any heteroatom or carbon atom.

Exemplary monocyclic heterocyclic groups include pyrrolidinyl; oxetanyl; pyrazolinyl; imidazolinyl; imidazolidinyl; oxazolyl; oxazolidinyl; isoxazolinyl; thiazolidinyl; isothiazolidinyl; tetrahydrofuryl; piperidinyl; piperazinyl; 2-oxopiperazinyl; 2-oxopiperidinyl; 2-oxopyrrolidinyl; 4-piperidonyl; tetrahydropyranyl; tetrahydrothiopyranyl; tetrahydrothiopyranyl sulfone; morpholinyl; thiomorpholinyl; thiomorpholinyl sulfoxide; thiomorpholinyl sulfone; 1,3-dioxolane; dioxanyl; thietanyl; thiiranyl; and the like. Exemplary bicyclic heterocyclic groups include quinuclidinyl; tetrahydroisoquinolinyl; dihydroisoindolyl; dihydroquinazolinyl (such as 3,4-dihydro-4-oxo-quinazolinyl); dihydrobenzofuryl; dihydrobenzothienyl; dihydrobenzothiopyranyl; dihydrobenzothiopyranyl sulfone; dihydrobenzopyranyl; indolinyl; isochromanyl; isoindolinyl; piperonyl; tetrahydroquinolinyl; and the like.

Substituted aryl, substituted heteroaryl, and substituted heterocycle may also be substituted with a second substituted-aryl, a second substituted-heteroaryl, or a second substituted-heterocycle to give, for example, a 4-pyrazol-1-yl-phenyl or 4-pyridin-2-yl-phenyl.

Designated numbers of carbon atoms (e.g., C₁₋₈) shall refer independently to the number of carbon atoms in an alkyl or cycloalkyl moiety or to the alkyl portion of a larger substituent in which alkyl appears as its prefix root.

Unless specified otherwise, it is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques known in the art as well as those methods set forth herein.

Where the compounds according to this invention have at least one stereogenic center, they may accordingly exist as enantiomers. Where the compounds possess two or more stereogenic centers, they may additionally exist as diastereomers. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

Some of the compounds of the present invention may have trans and cis isomers. In addition, where the processes for the preparation of the compounds according to the invention give rise to mixture of stereoisomers, these isomers may be separated by conventional techniques such as preparative chromatography. The compounds may be prepared as a single stereoisomer or in racemic form as a mixture of some possible stereoisomers. The non-racemic forms may be obtained by either synthesis or resolution. The compounds may, for example, be resolved into their components enantiomers by standard techniques, such as the formation of diastereomeric pairs by salt formation. The compounds may also be resolved by covalent linkage to a chiral auxiliary, followed by chromatographic separation and/or crystallographic separation, and removal of the chiral auxiliary. Alternatively, the compounds may be resolved using chiral chromatography.

This invention will be better understood by reference to the Experimental Details which follow, but those skilled in the art will readily appreciate that these are only illustrative of the invention as described more fully in the claims which follow thereafter. Additionally, throughout this application, various publications are cited. These publications describe more fully the state of the art to which this invention pertains.

### Experimental Details

### I. General Synthetic Schemes

Representative compounds of the present invention can be synthesized in accordance with the general synthetic methods described below and illustrated in the following general schemes. The products of some schemes can be used as intermediates to produce more than one of the instant compounds. The choice of intermediates to be used to produce subsequent compounds of the present invention is a matter of discretion that is well within the capabilities of those skilled in the art.

Procedures described in Schemes 1 to 7, wherein R₃ₐ, R_{3b}, R_{3c}, and R_{3d} are independently any R₃ group, and R₁, R₂, R₃, and R₄ are as described above, can be used to prepare compounds of the invention.

The substituted pyrimidines **1** can be prepared as shown in Scheme 1. The indanone or indandione **2** or the indene ester **3** can be condensed with an aldehyde to yield the substituted benzylidenes **4** (Bullington, J.L; Cameron, J.C.; Davis, J.E.; Dodd, J.H.; Harris, C.A.; Henry, J.R.; Pellegrino-Gensey, J.L.; Rupert, K.C.; Siekierka, J.J. Bioorg. Med. Chem. Lett. 1998, 8, 2489; Petrow, V.; Saper, J.; Sturgeon, B. J. Chem. Soc. 1949, 2134). This is then condensed with guanidine carbonate to form the indenopyrimidine **1.** 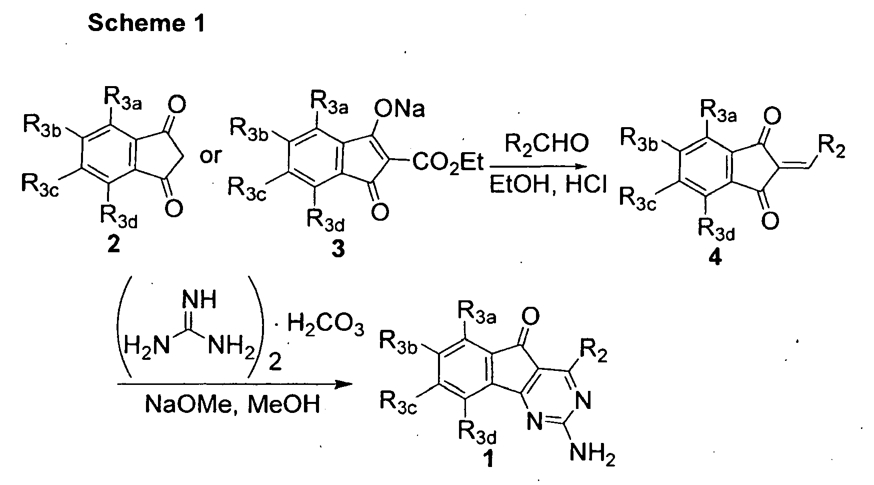 Alternatively, the pyrimidine compounds can be prepared as shown in Scheme 2. Sulfone **6** can be prepared by oxidation of the thiol ether **5** and the desired amines **7** can be obtained by treatment of the sulfone with aromatic amines.

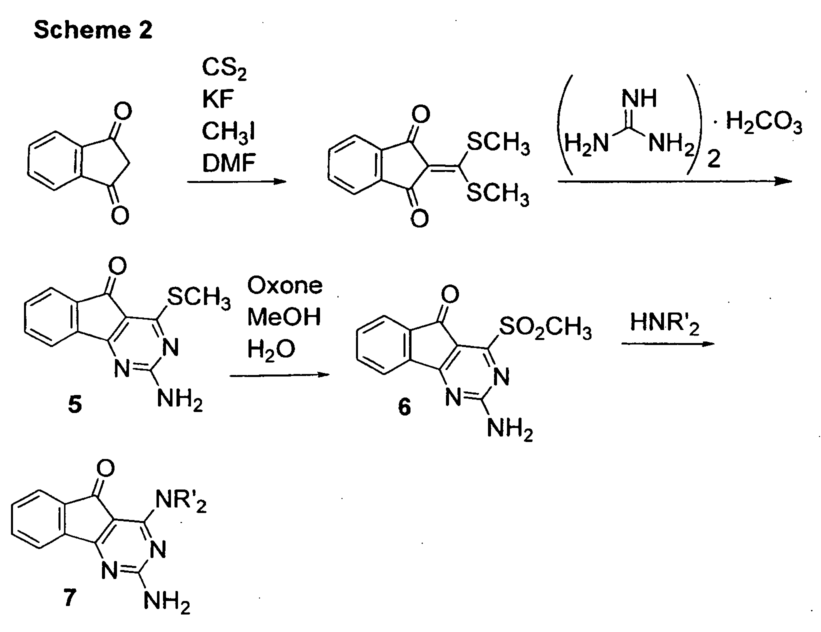

Pyrimidines with substituents on the fused aromatic ring could also be synthesized by the following procedure (Scheme 3). The synthesis starts with alkylation of furan with allyl bromide to provide 2-allylfuran. Diels-Alder reaction of 2-allylfuran with dimethylacetylene dicarboxylate followed by deoxygenation (Xing, Y.D.; Huang, N.Z. J. Org. Chem. 1982, 47, 140) provided the phthalate ester **8**. The phthalate.ester **8** then undergoes a Claisen condensation with ethyl acetate to give the styryl indanedione **9** after acidic workup (Buckle, D.R.; Morgan, N.J.; Ross, J.W.; Smith, H.; Spicer, B.A. J. Med. Chem. 1973, 16, 1334). The indanedione **9** is then converted to the dimethylketene dithioacetal **10** using carbon disulfide in the presence of KF. Addition of Grignard reagents to the dithioacetal **10** and subsequent reaction with guanidine provides the pyrimidines **11** as a mixture of isomers.

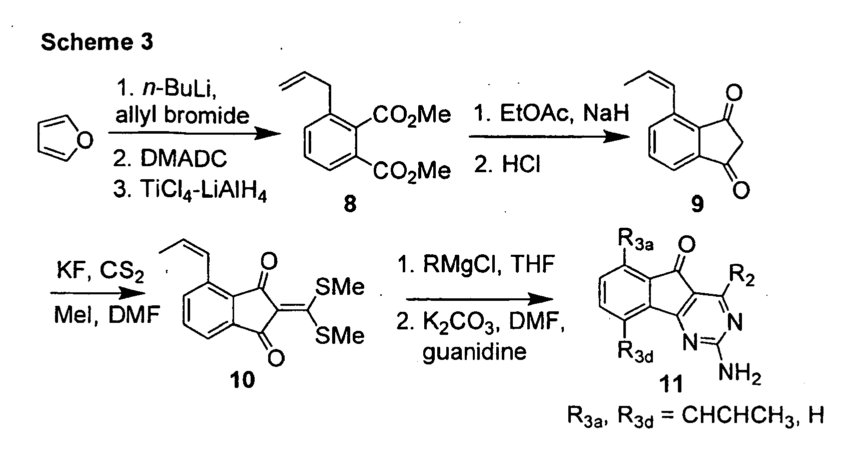

Dihydroxylation and oxidation give the aromatic aldehydes **13** that can be reductively aminated to provide amines **14.** The other isomer can be treated in a similar manner.

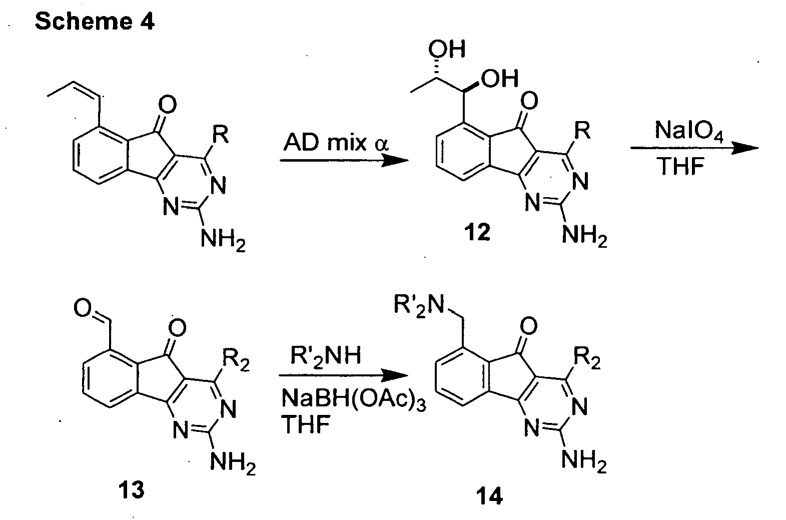

3-Dicyanovinylindan-1-one **(15)** (Scheme 5) was obtained using the published procedure (Bello, K.A.; Cheng, L.; Griffiths, J. J. Chem. Soc., Perkin Trans. II 1987, 815). Reaction of 3-dicyanovinylindan-1-one with an aldehyde in the presence of ammonium hydroxide produced dihydropyridines **16** (EI-Taweel, F.M.A.; Sofan, M.A.; E.-Maati, T.M.A.; Elagamey, A.A. Boll. Chim. Farmac. 2001, 140, 306). These compounds were then oxidized to the corresponding pyridines **17** using chromium trioxide in refluxing acetic acid.

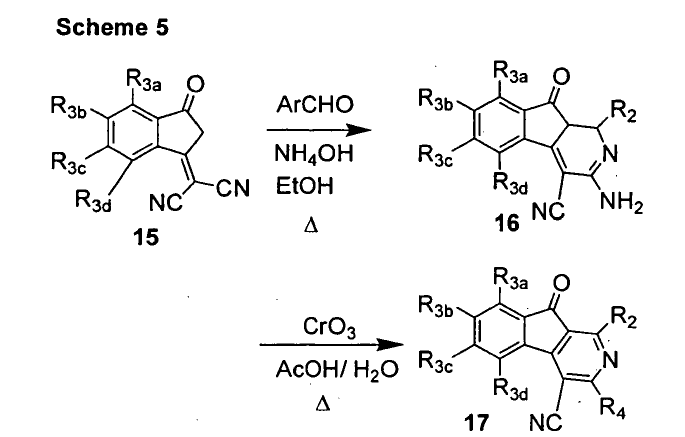

The ketone of pyridines **17** can be reduced to provide the benzylic alcohols **18.** Alternatively, the nitriles can be hydrolyzed with sodium hydroxide to give the carboxylic acids **19** (Scheme 6).

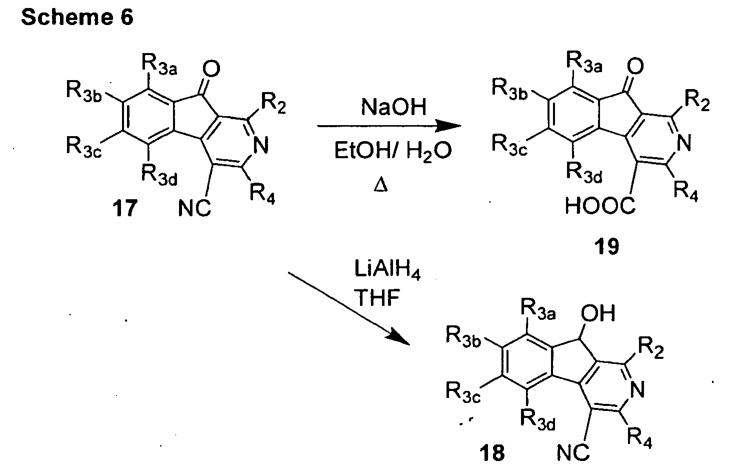

The acids can then be converted to carboxylic esters **20** or amides **21** using a variety of methods. In general, the esters **20** are obtained by treatment with silver carbonate followed by an alkyl chloride or by coupling with diethylphosphoryl cyanide (DEPC) and the appropriate alcohol (Okawa, T.; Toda, M.; Eguchi, S.; Kakehi, A. Synthesis 1998, 1467). The amides **21** are obtained by coupling the carboxylic acid with the appropriate amine in the presence of DEPC or 1-(3-dimethylaminopropyl)-3-ethylcarbodiimide hydrochloride (EDCI). Esters **20** can also be obtained by first reacting the carboxylic acids **19** with a dibromoalkane followed by displacement of the terminal bromide with an amine (Scheme 7).

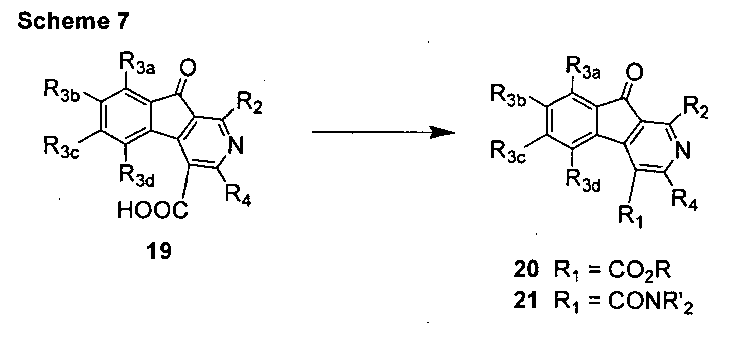

### II. Specific Compound Syntheses

Specific compounds which are representative of this invention can be prepared as per the following examples. No attempt has been made to optimize the yields obtained in these reactions. Based on the following, however, one skilled in the art would know how to increase yields through routine variations in reaction times, temperatures, solvents and/or reagents.

The products of certain syntheses can be used as intermediates to produce more than one of the instant compounds. In those cases, the choice of intermediates to be used to produce compounds of the present invention is a matter of discretion that is well within the capabilities of those skilled in the art.

### EXAMPLE 1

### Synthesis of Benzylidene 4 (R₂ = 2-furyl, R₃ₐ = F, R_{3b}, R_{3c},R_{3d} = H)

A mixture of **3** (3.0 g, 11.69 mmol) and 2-furaldehyde (1.17 g, 12.17 mmol) in 75 mL of ethanol and 3 mL of concentrated hydrogen chloride was allowed to stir at reflux for 16 hours. The reaction was then cooled to room temperature, and the resulting precipitate was filtered off, washed with ethanol, diethyl ether, and air dried to afford 1.27 g (45%) of product.

### EXAMPLE 2

### Synthesis of Indenopyrimidine 1 (R₂ = 2-furyl, R3a = F, R_{3b}, R_{3c},R_{3d} = H

A mixture of **4** (0.5 g, 2.06 mmol), guanidine carbonate (0.93 g, 5.16 mmol), and 20.6 mL of 0.5 M sodium methoxide in methanol was stirred at reflux for 16 hours. The reaction mixture was cooled to room temperature, and diluted with water. The resulting precipitate was collected, washed with water, ethanol, diethyl ether, and then dried. Crude material was then purified over silica gel to afford 0.024 g (4%) of product. MS m/z 282.0 (M+H).

### EXAMPLE 3

### Synthesis of 2-Amino-4-methanesulfonyl-indeno[1,2-d]pyrimidin-5-one

To a suspension of **5** (Augustin, M.; Groth, C.; Kristen, H.; Peseke, K.; Wiechmann, C. J. Prakt. Chem. 1979, 321, 205) (1.97 g, 8.10 mmol) in MeOH (150 mL) was added a solution of oxone (14.94 g, 24.3 mmol) in H₂O (100 mL). The mixture was stirred at room temperature overnight then diluted with cold H₂O (500 mL), made basic with K₂CO₃ and filtered. The product was washed with water and ether to give 0.88 g (40%) of sulfone 6. MS m/z 297.9 (M+Na).

### EXAMPLE 4

### Synthesis of Aminopyrimidine 7 (R₂ = NHPh, R₃ = H)

A mixture of sulfone **6** (0.20 g, 0.73 mmol) and aniline (0.20 g, 2.19 mmol) in N-methylpyrrolidinone (3.5 mL) was heated to 100 °C for 90 minutes. After cooling to room temperature, the mixture was diluted with EtOAc (100 mL), washed with brine (2 x 75 mL) and water (2 x 75 mL), and dried over Na₂SO₄. After filtration and concentration in vacuo, the residue was purified by column chromatography eluting with 0-50% EtOAc in hexane to yield 0.0883 g (42%) of product 7. MS m/z 289.0 (M+H).

### EXAMPLE 5

### Synthesis of Phthalate Ester 8

A 1.37 M hexanes solution of n-BuLi (53.6 mL, 73.4 mmol) was added to a cold, -78°C, THF solution (100 mL) of furan (5.3 mL, 73.4 mmol) and the reaction was then warmed to 0 °C. After 1.25 h at 0°C neat allyl bromide (7.9 mL, 91.8 mmol) was added in one portion. After 1 h at 0°C, saturated aqueous NH₄Cl was added and the layers were separated. The aqueous phase was extracted with EtOAc and the combined organics were washed with water and brine, dried over Na₂SO₄, and concentrated to give 4.6 g (58%) of 2-allylfuran which was used without further purification.

The crude allyl furan (4.6 g, 42.6 mmol) and dimethylacetylene dicarboxylate (5.2 mL, 42.6 mmol) were heated to 90°C in a sealed tube without solvent. After 6 h at 90°C the material was cooled and purified by column chromatography eluting with 25% EtOAc in hexanes to give 5.8 g (54%) of the oxabicycle as a yellow oil. MS m/z 251 (M+H).

Tetrahydrofuran (60 mL) was added dropwise to neat TiCl₄ (16.5 mL, 150.8 mmol) at 0°C. A 1.0 M THF solution of LiAlH₄ (60.3 mL, 60.3 mmol) was added dropwise, changing the color of the suspension from yellow to a dark green or black suspension. Triethylamine (2.9 mL, 20.9 mmol) was added and the mixture was refluxed at 75-80°C. After 45 min, the solution was cooled to rt and a THF solution (23 mL) of the oxabicycle (5.8 g, 23.2 mmol) was added to the dark solution. After 2.5 h at rt, the solution was poured into a 20% aq. K₂CO₃ solution (200 mL) and the resulting suspension was filtered. The precipitate was washed several times with CH₂Cl₂ and the filtrate layers were separated. The aqueous phase was extracted with CH₂Cl₂ and the combined organics were washed with water and brine, dried over Na₂SO₄, concentrated, and purified by column chromatography eluting with 25 % EtOAc in hexanes to give 3.5 g (64%) of the phthalate ester **8** as a yellow oil. MS m/z 235 (M+H).

### EXAMPLE 6

### Synthesis of Indanedione 9

A 60% dispersion of sodium hydride in mineral oil (641 mg, 16.0 mmol) was added to an EtOAc solution (3.5 mL) of the phthalate ester **8** (2.5 g, 10.7 mmol), and the resulting slurry was refluxed. After 1 h the solution became viscous so an additional 7.5 mL of EtOAc was added. After 4 h at reflux the suspension was cooled to rt and filtered to give a yellow solid. This solid was added portionwise to a solution of HCl (25 mL water and 5 mL conc. HCl) at 80°C. The suspension was heated for an additional 30 min at 80°C, cooled to rt, and filtered to give 1.2 g (60%) of the indanedione **9** as a yellow solid. MS m/z 187 (M+H).

### EXAMPLE 7

### Synthesis of Dimethylketene Dithioacetal 10

Solid potassium fluoride (7.5 g, 129.1 mmol) was added to a 0°C solution of indanedione **9** (1.2 g, 6.5 mmol) and CS₂ (0.47 mL, 7.8 mmol) in DMF (10 mL). The cold bath was removed and after 30 min neat iodomethane (1.00 mL, 16.3 mmol) was added. After 5 h at rt, the suspension was diluted with EtOAc and then washed with water and brine. The organic layer was dried over Na₂SO₄, concentrated, and purified by column chromatography eluting with 20 % EtOAc in hexanes to give 1.4 g (75%) of the dimethylketene dithioacetal **10** as a yellow solid. MS m/z 291 (M+H).

### EXAMPLE 8

### Synthesis of Pyrimidine 11 (R₂=Ph, R₃ₐ=CHCHCH₃, R_{3d}=H)

A 2.0 M solution of PhMgCl in THF (13 mL, 25.7 mmol) was added to a -78°C solution of dimethylketene dithioacetal **10** (5.7 g, 19.8 mmol) in 200 mL of THF. After 3 h at-78°C, saturated aqueous NH₄CI was added and the layers were separated. The aqueous layer was extracted with EtOAc and the combined organic extracts were washed with water and brine, dried over Na₂SO₄, concentrated, and purified by column chromatography eluting with 20 % EtOAc in hexanes to give 4.9 g (77%) of the thioenol ether as a yellow solid. MS m/z 321 (M+H).

Solid guanidine hydrochloride (1.5 g, 15.3 mmol) was added to a solution of the thioenol ether (4.9 g, 15.3 mmol) and K₂CO₃ (2.6 g, 19.1 mmol) in 30 mL of DMF and the solution was heated to 80°C. After 6 h at 80°C, the solution was diluted with EtOAc and washed with water and brine. The organic layer was dried over Na₂SO₄, concentrated, and purified by column chromatography eluting with 40 % EtOAc in hexanes to give 4.6 g (96%) of the pyrimidine regioisomers **11** as yellow solids. MS m/z 314 (M+H).

### EXAMPLE 9

### Synthesis of Aldehyde 13 (R₂=Ph)

Solid MeSO₂NH₂ (277 mg, 2.9 mmol) was added to a t-BuOH:H₂O (1:1) solution (30 mL) of AD-mix-α (4.0 g). The resulting yellow solution was added to an EtOAc solution (15 mL) of the pyrimidine (910 mg, 2.9 mmol). After 3 days, solid sodium sulfite (4.4 g, 34.9 mmol) was added. After stirring for 1.5 h, the heterogeneous solution was diluted with EtOAc and the layers were separated. The aqueous phase was extracted with EtOAc and the combined extracts were washed with water and brine, dried over Na₂SO₄, concentrated, and purified by column chromatography eluting with 100 % EtOAc to give 710 mg (70%) of the intermediate diol **12**. MS m/z 348 (M+H).

Solid HlO₄-2H₂O (933 mg, 4.1 mmol) was added to a 0 °C solution of diol **12** (710 mg, 2.1 mmol) in THF. After 1.5 h at 0°C, the solution was diluted with EtOAc and the organic phase was washed with saturated aqueous NaHCO₃, water, and brine. The organic layer was dried over Na₂SO₄ and concentrated to give 603 mg (98%) of aldehyde **13** as a yellow solid that was used without further purification. MS m/z 302 (M+H).

### EXAMPLE 10

### Synthesis of Amine 14 via Reductive Amination (R₃ₐ= N(-CH₂CH₂OCH₂CH₂-)

Solid NaBH(OAc)₃ (53 mg, 0.25 mmol) was added to a solution of aldehyde **13** (50 mg, 0.17 mmol), morpholine (0.034 mL, 0.34 mmol), and AcOH (0.014 mL, 0.25 mmol) in 1 mL of THF. After 3 d the solution was filtered and concentrated. The resulting material was dissolved in CH₂Cl₂ and washed with saturated aqueous NaHCO₃ and brine, dried over Na₂SO₄, concentrated, and purified by column chromatography eluting with 0-10 % MeOH in CH₂Cl₂ to give 38 mg (60%) of the amine **14** as a yellow solid. MS m/z 373 (M+H). The product was dissolved in a minimum amount of CH₂Cl₂. and treated with 1.0 M HCl in ether to obtain the hydrochloride salt.

### EXAMPLE 11

### Cyclization to Form Dihydropyridine 16 (R₂ = 2-furyl, R₃=H)

To a solution of 3-dicyanovinylindan-1-one (4.06 g, 20.9 mmol) in 200 mL of ethanol was added 2-furaldehyde (3.01 g, 31.4 mmol) and 25 mL of conc. NH₄OH. The solution was heated to reflux for 2 h and allowed to cool to rt overnight. The mixture was concentrated in vacuo to remove ethanol. The residue was filtered and washed with water. The purple solid obtained was dried to yield 5.92 g (89%). MS m/z 290 (M⁺+1).

### EXAMPLE 12

### Oxidation of Dihydropyridine 16 to Pyridine 17 (R₂ = 2-furyl. R₃ = H, R₄ = NH₂, R₅ = CN, X = O)

To a refluxing solution of dihydropyridine **16** (5.92 g, 20.4 mmol) in acetic acid (100 mL) was added a solution of chromium (VI) oxide (2.05 g, 20.4 mmol) in 12 mL of water. After 10 minutes at reflux, the reaction was diluted with water until a precipitate started to form. The mixture was cooled to room temperature and filtered. The residue was washed with water to give 4.64 g (79%) of a brown solid. MS m/z 288 (M⁺+1).

### EXAMPLE 13

### Reduction of Ketone 17 to Alcohol 18 (R₂ = 2-furyl, R₃ = H, R₄ = NH₂, R₅ = CN, X = H, OH)

To a 0°C solution of ketone **17** (0.115 g, 0.40 mmol) in 12 mL of THF was added a 1.0 M LiAlH₄ solution in THF (0.40 mL, 0.40 mmol). The reaction was stirred at 0°C for 1 h. The reaction was quenched by the addition of ethyl acetate (1.5 mL), water (1.5 mL), 10% aq. NaOH (1.5 mL), and saturated aq. NH₄Cl (3.0 mL). The mixture was extracted with ethyl acetate (3 x 35 mL), washed with brine, and dried over sodium sulfate. The remaining solution was concentrated to yield 0.083 g (72%) of a yellow solid. MS m/z 290 (M⁺+1).

### EXAMPLE 14

### Hydrolysis of Nitrile 17 to Carboxylic Acid 19 (R₂ = 2-furyl, R₃ = H, R₄ = NH2_{,} R₅ = COOH, X = O)

To a mixture of nitrile **17** (0.695 g, 2.42 mmol) and ethanol (30 mL) was added 5 mL of 35% aqueous sodium hydroxide. The resulting mixture was heated to reflux overnight. After cooling to rt, the solution was poured into water and acidified with 1 N HCI. The resulting precipitate was isolated by filtration and washed with water to yield 0.623 g (84%) of a brown solid. MS m/z 329 (M⁺+23).

### EXAMPLE 15

### Synthesis of Carboxylic Ester 20 with Silver Carbonate (R₂ = 2-furyl, R₃ = H, R₄ = NH₂, R₅ = CO₂CH₂CH₂NMe₂, X = O)

A suspension of carboxylic acid **19** (5.0 g, 16.3 mmol), silver carbonate (5.8 g, 21.2 mmol), and tetrabutylammonium iodide (1.5 g, 4.1 mmol) in 80 mL of DMF was heated to 90°C. After 1 h, the mixture was cooled to rt and 2-(dimethylamino)ethylchloride hydrochloride (2.4 g, 16.3 mmol) was added and the mixture was heated to 100°C. After 7 h, the reaction was filtered while hot, concentrated and purified by column chromatography eluting with 0-10% MeOH/CH₂Cl₂ to yield 0.160 g (3%) of a yellow solid. MS m/z 378 (M⁺+1). The product was dissolved in a minimum of dichloromethane and treated with 1.0 M HCI in ether to obtain the hydrochloride salt.

### EXAMPLE 16

### Synthesis of Carboxylic Ester 20 with DEPC (R₂ = 2-furyl, R₃ = H, R₄ = NH₂, R₅ = CO₂CH₂CH(-CH₂CH₂CH₂(Me)N-), X=O)

To a mixture of carboxylic acid **19** (0.40 g, 1.3 mmol) and (S)-1-methyl-2-pyrrolidinemethanol (0.50 mL, 3.9 mmol) in DMF (30 mL) was added 0.20 mL (1.3 mmol) of diethylphosphoryl cyanide and triethylamine (0.20 mL, 1.3 mmol). The reaction was stirred at 0°C for one hour and then heated up to approximately 70°C overnight. The reaction was then cooled to rt and diluted with ethyl acetate. The organic mixture was washed with saturated aqueous NaHCO₃, water, and brine. After being dried with sodium sulfate, the solution was concentrated. The residue was purified by column chromatography eluting with 10-100% ethyl acetate in hexane and then preparative TLC eluting with 2% MeOH in dichloromethane to yield 1.9 mg (0.4%) of a yellow solid. MS m/z 404 (M⁺+1).

### EXAMPLE 17

### Synthesis of Carboxylic Amide 21 with DEPC (R₂ = 2-furyl, R₃ = H, R₄ = NH₂, R₅ = CO₂CH₂CH(-CH₂CH₂CH₂(Me)N-), X = O)

To a mixture of carboxylic acid **19** (0.25 g, 0.82 mmol) and N,N,N'-trimethylethylenediamine (0.14 mL, 1.08 mmol) in DMF (20 mL) was added 0.12 mL (0.82 mmol) of diethylphosphoryl cyanide and triethylamine (0.11 mL, 0.82 mmol). The reaction was stirred at 0°C for one hour and then heated up to approximately 60°C overnight. The reaction was then cooled to rt and diluted with ethyl acetate. The organic mixture was washed with saturated aqueous NaHCO₃, water, and brine. After being dried with magnesium sulfate, the solution was concentrated. The residue was purified by column chromatography eluting with 0-10% methanol in dichloromethane and then preparative TLC eluting with 1% MeOH in dichloromethane to yield 3.3 mg (10%) of a yellow solid. MS m/z 391 (M⁺+1). The product was dissolved in a minimum of diethyl ether and treated with 1.0 M HCI in ether to obtain the hydrochloride salt.

### EXAMPLE 18

### Synthesis of Carboxylic Amide 21 with EDCI (R₂ = 2-furyl, R₃ = H, R₄ =NH₂, R₅ = CON(-CH₂CH₂NMeCH₂CH₂-), X = O)

A mixture of carboxylic acid **19** (0.300 g, 0.979 mmol), N-methylpiperazine (0.295 g, 2.94 mmol), 1-(3-dimethylaminopropyl)-3-ethylcarbodiimide hydrochloride (0.563 g, 2.94 mmol) 1-hydroxybenzotriazole hydrate (0.397 g, 2.94 mmol), triethylamine (0.298 g, 2.94 mmol) in DMF (8 mL) was stirred at rt overnight. The mixture was then diluted with water and extracted several times with ethyl acetate. The combined organics were washed twice with brine and then dried over sodium sulfate. The solution was concentrated and then purified by column chromatography to afford 0.092 g (2%) of solid. MS m/z 389 (M⁺+1). The product was treated with 1.0 M HCI in ether to obtain the hydrochloride salt.

### EXAMPLE 19

### Synthesis of Carboxylic Ester 20 via a dibromoalkane (R₂ = Ph, R₃ = H, R₄ = NH₂, R₅ = CO₂CH₂ CH₂CH₂NMe₂, X = O)

To a solution of carboxylic acid **19** (0.100 g, 0.32 mmol) in DMF (1.5 mL) was added 60% NaH dispersion in mineral oil (0.013 g, 0.32 mmol). After 10 min at rt, 1,3-dibromopropane (0.035 mL, 0.35 mmol) was added and the solution was stirred at rt for 17 h. After concentration, the residue was purified via column chromatography eluting with 40% ethyl acetate in hexanes to yield 0.014 g (9%) of a yellow solid. MS m/z 437 (M⁺+1).

To a solution of the yellow solid (0.014 mg, 0.03 mmol) in a sealed tube was added a 40% aqueous solution of dimethylamine (0.5 mL, 3.0 mmol). The tube was heated to 75°C for 2 h before concentrating. The residue was purified by column chromatography eluting with 0-10% methanol in dichloromethane to yield 0.009 g (70%) of a yellow solid. MS m/z 402 (M⁺+1). The productwas dissolved in a minimal amount of CH₂Cl₂ and treated with 1 N HCI in ether to obtain the hydrochloride salt.

Following the general synthetic procedures outlined above and in Examples 1-19, the compounds of Table 1 below were prepared.

**Table 1**

| | | | | | | | | |
|---|---|---|---|---|---|---|---|---|
| 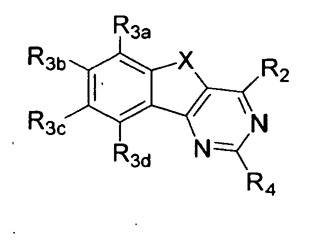 | | | | | | | | |

| No. | R₂ | R₃ₐ | R_{3b} | R_{3c} | R_{3d} | R₄ | X | MS (M+1) |
|---|---|---|---|---|---|---|---|---|
| 1 | 4-MeOPh | H | H | H | H | NH₂ | CH₂ | 290 |
| 2 | 4-MeOPh | H | H | H | H | NH₂ | CO | 304 |
| 3 | 2-furyl | H | H | H | H | CH₂ | CO | 264 |
| 4 | 2-furyl | H | H | H | H | NH₂ | CH₂ | 250 |
| 5 | 3-pyridyl | H | H | H | H | NH₂ | CO | 297 (+Na) |
| 6 | 4-pyridyl | H | H | H | H | NH₂ | CO | 275 |
| 7 | 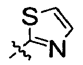 C₃H₂NS | H | H | H | H | CH₂ | CO | 281 |
| 8 | 4-ClC₆H₄ | H | H | H | H | CH₂ | CO | 308 |
| 9 | 3-NO₂C₆H₄ | H | H | H | H | NH₂ | CO | 319 |
| 10 | Ph | H | H | H | H | NH₂ | CO | 274 |
| 11 | 3-MeOC₆H₄ | H | H | H | H | NH₂ | CO | 304 |
| 12 | 2-MeOC₆H₄ | H | H | H | H | NH₂ | CO | 304 |
| 13 | 3-HOC₆H₄ | H | H | H | H | NH₂ | CO | 290 |
| 14 | 2-thiophenyl | H | H | H | H | NH₂ | CO | 302 |
| 15 | 3-thiophenyl | H | H | H | H | NH₂ | CO | 302 |
| 16 | 2-furyl | H | Br | H | H | NH₂ | CO | 342 |
| 17 | 2-furyl | OH | H | H | H | NH₂ | CO | 280 |
| 18 | SCH3 | NH₂ | H | H | H | NH₂ | CO | 259* |
| 19 | 3-FC₆H₄ | H | H | H | H | NCHNMe₂ | CO | 347 |
| 20 | 2-furyl | NH₂ | H | H | H | NH₂ | CO | 279 |
| 21 | 2-furyl | H | H | H | NH₂ | NH₂ | CO | 279 |
| 22 | 2-furyl | H | CF₃ | H | H | NH2 | CO | 332 |
| 23 | 2-furyl | H | H | CF₃ | H | NH₂ | CO | 332 |
| 24 | Ph | H | H | H | H | NHMe | CO | 288 |
| 25 | 2-furyl | H | Cl | Cl | H | NH₂ | CO | 332 |
| 26 | 2-furyl | Cl | H | H | Cl | NH₂ | CO | 332 |
| 27 | Ph | H | H | H | H | N(CH₇)₂NEt₂ | CO | 373 |
| 28 | 3,4-F₂C₆H₃ | H | H | H | H | NH₂ | CO | 310 |
| 29 | 3,5-F₂C₆H₃ | H | H | H | H | NH₂ | CO | 310 |
| 30 | 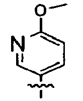 C₆H₆NO | H | H | H | H | NH₂ | CO | 305 |
| 31 | 3,4,5-F₃C₂H₂ | H | H | H | H | NH₂ | CO | 340 (M+Na) |
| | | 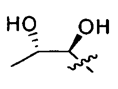 | | | | | | |
| 32 | Ph | C₃H₇O₂ | H | H | H | NH₂ | CO | 348 |
| | | | | | 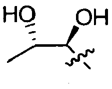 | | | |
| 33 | Ph | H | H | H | C₃H₇O₂ | NH₂ | CO | 348 |
| 34 | 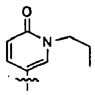 C₈H₁₀NO | H | H | H | H | NH₂ | CO | 333 |
| 35 | 2-furyl | H | H | Br | H | NH₂ | CO | 342/344 |
| 36 | 2-furyl | H | H | H | F | NH₂ | CO | 282 |
| 37 | 2-furyl | MeO | H | H | H | NH₂ | CO | 294 |
| 38 | 4-FC₆H₄ | H | H | H | H | NH₂ | CO | 292 |
| 39 | 3-FC₆H₄ | H | H | H | H | NH₂ | CO | 292 |
| 40 | SO₂Me | H | H | H | H | NH₂ | CO | 298 * |
| 41 | Sme | H | H | H | H | NH₂ | CO | 266* |
| 42 | Ome | H | H | H | H | NH₂ | CO | 477 (2M+Na) * |
| 43 | NHPh | H | H | H | H | NH₂ | CO | 289 |
| 44 | 3-furyl | H | H | H | H | NH₂ | CO | 264 |
| 45 | 5-methyl-2-furyl | H | H | H | H | NH2 | CO | 278 |
| 46 | 2-furyl | OCH₂CH₂NHCO₂*t*Bu | H | H | H | NH₂ | CO | 437 |
| 47 | Ph | H | H | H | H | Me | CO | 297 |
| 48 | Ph | H | H | H | H | OMe | CO | 291 |
| 49 | Ph | CH₂NMeCH₂CH₂NMe₂ | H | H | H | NH₂ | CO | 388 |
| 50 | Ph | 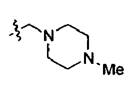 C₆H₁₃N₂ | H | H | H | NH₂ | CO | 386 |
| | | 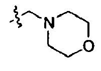 | | | | | | |
| 51 | Ph | C₅H₁₀NO | H | H | H | NH₂ | CO | 373 |
| 52 | Ph | CH₂NEt₂ | H | H | H | NH₂ | CO | 359 |
| | | 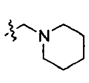 | | | | | | |
| 53 | Ph | C₆H₁₂N | H | H | H | NH₂ | CO | 371 |
| 54 | Ph | 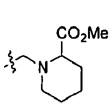 | H | H | H | NH₂ | CO | 429 |
| | | C₈H₁₄NO₂ | | | | | | |
| 55 | Ph | 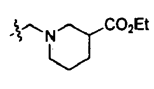 C₉H₁₆NO₂ | H | H | H | NH₂ | CO | 443 |
| 56 | Ph | CH₂NMeCH₂CO₂Me | H | H | H | NH₂ | CO | 389 |
| 57 | Ph | 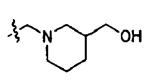 C₇H₁₄NO416 | H | H | H | NH₂ | CO | 401 |
| 58 | Ph | | H | H | H | NH₂ | CO | 416 |
| 59 | Ph | 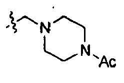 C₇H₁₃N₂O | H | H | H | NH₂ | CO | 414 |
| 60 | Ph | 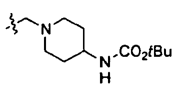 C₁₁H₂₁N₂O₂ | H | H | H | NH₂ | CO | 486 |
| 61 | Ph | 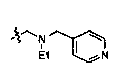 C₉H₁₃N₂ | H | H | H | NH₂ | CO | 422 |
| 62 | Ph | 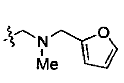 C₇H₁₀NO | H | H | H | NH₂ | CO | 397 |

| | | | | | | | | |
|---|---|---|---|---|---|---|---|---|
| * SYNTHESIS EXAMPLE ONLY | | | | | | | | |

**Table 2**

| | | | | | | | | |
|---|---|---|---|---|---|---|---|---|
| 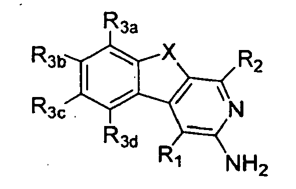 | | | | | | | | |

| No | X | R₂ | R₃ₐ | R_{3b} | R_{3c} | R_{3d} | R₁ | MS (M+1) |
|---|---|---|---|---|---|---|---|---|
| 63 | CO | 2-furyl | H | H | H | H | CN | 288 |
| 64 | CO | Ph | H | H | H | H | CN | 298 |
| 65 | CO | Ph | H | H | H | H | COOH | 315 (M-1) |
| 66 | CO | 3-furyl | H | H | H | H | CN | 288 |
| 67 | CO | 3-FC₆H₄ | H | H | H | H | CN | 316 |
| 68 | CO | 3-pyridyl | H | H | H | H | CN | 299 |
| 69 | CO | 2-furyl | H | H | H | H | COOH | 305 (M-1) |
| 70 | CO | 2-furyl | H | H | H | H | CO₂CH₂CH₂NMe₂ | 378 |
| 71 | CO | 4-FC₆H₄ | H | H | H | H | CN | 316 |
| 72 | CO | 2-thiophenyl | H | H | H | H | CN | 304 |
| 73 | CO | 3-thiophenyl | H | H | H | H | CN | 304 |
| 74 | CO | 3-MeOC₆H₄ | H | H | H | H | CN | 328 |
| 75 | CO | 2-imidazolyl | H | H | H | H | CN | 288 |
| 76 | CO | 2-furyl | H | H | H | H | CONHCH₂CH₂NMe₂ | 377 |
| 77 | CO | 2-furyl | H | H | H | H | CONMeCH₂CH₂NMe₂ | 391 |
| 78 | CO | 2-furyl | H | H | H | H | CONHCHMeCH₂NMe₂ | 391 |
| 79 | CO | 2-furyl | F | F | F | F | CN | 358 (M-1) |
| | | | | | | | 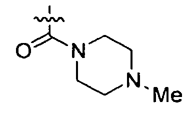 | |
| 80 | CO | 2-furyl | H | H | H | H | C₆H₄NO₂C₆H₁₁N₂O | 389 |
| 81 | CO | Ph | H | H | H | H | CO₂CH₂CH₂NMe₂ | 388 |
| 82 | CO | 2-furyl | H | H | H | H | 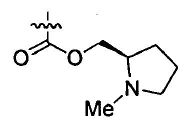 C₇H₁₂NO₂ | 404 |
| 83 | CO | Ph | H | H | H | H | 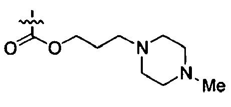 C₉H₁₇N₂O₂ | 457 |
| 84 | CO | Ph | H | H | H | H | 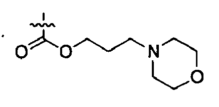 C₈H₁₄NO₃ | 444 |
| 85 | CO | Et | H | H | H | H | CN | 250 |
| 86 | CO | *i*-Bu | H | H | H | H | CN | 278 |
| 87 | CO | Ph | H | H | H | H | CO₂CH₂CH₂CH₂NMe₂ | 402 |
| 88 | CO | Ph | H | H | H | H | 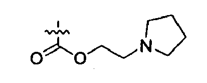 C₇H₁₂NO₂ | 414 |
| 89 | CHOH | 2-furyl | H | H | H | H | CN | 290 |
| 90 | CO | Ph | H | H | H | H | 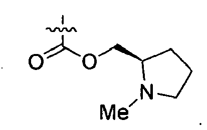 C₇H₁₂NO₂ | 414 |
| 91 | CO | Ph | H | H | H | H | 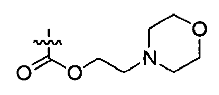 C₇H₁₂NO₂ | 430 |
| 92 | CO | Ph | H | H | H | H | CO₂CH₂CHMeCH₂NMe₂ | 416 |
| 93 | CO | 3-thiophenyl | H | H | H | H | CO₂CH₂CH₂NMe₂ | 394 |
| 94 | CO | CH₂CH₂CHCH₂ | H | H | H | H | CN | 276 |
| 95 | CO | c-Hex | H | H | H | H | CN | 302 (M-1) |
| 96 | CO | 2-furyl | H | H | H | H | (S)-CO₂CHMeCH₂NMe₂ | 392 |

### III. Biological Assays and Activity

### Ligand Binding Assay for Adenosine A2a Receptor

Ligand binding assay of adenosine A2a receptor was performed using plasma membrane of HEK293 cells containing human A2a adenosine receptor (PerkinElmer, RB-HA2a) and radioligand [³H]CGS21680 (PerkinElmer, NET1021). Assay was set up in 96-well polypropylene plate in total volume of 200 µl by sequentially adding 20 µL1:20 diluted membrane, 130 µLassay buffer (50 mM Tris·HCl, pH7.4 10 mM MgCl₂, 1 mM EDTA) containing [³H] CGS21680, 50 µL diluted compound (4X) or vehicle control in assay buffer. Nonspecific binding was determined by 80 mM NECA. Reaction was carried out at room temperature for 2 hours before filtering through 96-well GF/C filter plate pre-soaked in 50 mM Tris·HCl, pH7.4 containing 0.3% polyethylenimine. Plates were then washed 5 times with cold 50 mM Tris·HCl, pH7.4, dried and sealed at the bottom. Microscintillation fluid 30 µl was added to each well and the top sealed. Plates were counted on Packard Topcount for [³H]. Data was analyzed in Microsoft Excel and GraphPad Prism programs. (Varani, K.; Gessi, S.; Dalpiaz, A.; Borea, P.A. British Journal of Pharmacology, 1996, 117, 1693)

### Adenosine A2a Receptor Functional Assay

CHO-K1 cells overexpressing human adenosine A2a receptors and containing cAMP-inducible beta-galactosidase reporter gene were seeded at 40-50K/well into 96-well tissue culture plates and cultured for two days. On assay day, cells were washed once with 200 µL assay medium (F-12 nutrient mixture/0.1 % BSA). For agonist assay, adenosine A2a receptor agonist NECA was subsequently added and cell incubated at 37°C, 5% CO₂ for 5 hrs before stopping reaction. In the case of antagonist assay, cells were incubated with antagonists for 5 minutes at R.T. followed by addition of 50 nM NECA. Cells were then incubated at 37°C, 5% CO₂ for 5 hrs before stopping experiments by washing cells with PBS twice. 50 µL 1X lysis buffer (Promega, 5X stock solution, needs to be diluted to 1X before use) was added to each well and plates frozen at -20°C. For β-galactosidase enzyme colorimetric assay, plates were thawed out at room temperature and 50 µL 2X assay buffer (Promega) added to each well. Color was allowed to develop at 37°C for 1 h or until reasonable signal appeared. Reaction was then stopped with 150 µL 1 M sodium carbonate. Plates were counted at 405 nm on Vmax Machine (Molecular Devices). Data was analyzed in Microsoft Excel and GraphPad Prism programs. (Chen, W.B.; Shields, T.S.; Cone, R. D. Analytical Biochemistry, 1995, 226, 349; Stiles, G. Journal of Biological Chemistry, 1992,267,6451)

### Haloperidol-induced catalepsy study in C57bl/6 mice

Mature male C57bl/6 mice (9-12 week old from ACE) were housed two per cage in a rodent room. Room temperature was maintained at 64-79 degrees and humidity at 30-70% and room lighting at 12 hrs light/12 hrs dark cycle. On the study day, mice were transferred to the study room. The mice were injected subcutaneously with haloperidol (Sigma H1512, 1.0 mg/ml made in 0.3% tartaric acid, then diluted to 0.2 mg/ml with saline) or vehicle at 1.5 mg/kg, 7.5ml/kg. The mice were then placed in their home cages with access to water and food. 30 minutes later, the mice were orally dosed with vehicle (0.3% Tween 80 in saline) or compounds at 10 mg/kg, 10 ml/kg (compounds, 1mg/ml, made in 0.3% Tween 80 in saline, sonicated to obtain a uniform suspension). The mice were then placed in their home cages with access to water and food. 1 hour after oral dose, the catalepsy test was performed. A vertical metal-wire grid (1.0 cm squares) was used for the test. The mice were placed on the grid and given a few seconds to settle down and their immobility time was recorded until the mice moved their back paw(s). The mice were removed gently from the grid and put back on the grid and their immobility time was counted again. The measurement was repeated three times. The average of three measurements was used for data analysis.

Compound 70 showed 87% inhibition and compound 3 showed 90% inhibition of haloperidol-induced catalepsy when orally dosed at 10 mg/kg.

**Table 5**

| No. | Ki (nM) | | |
|---|---|---|---|
| | A2a binding | A2a antagonist function | A1 antagonist function |
| 1 | 44.64 | 233.7 | 52.98 |
| 2 | 2.032 | 6.868 | 5.32 |
| 3 | 0.26 | 0.0066 | 0.288 |
| 4 | 0.885 | 2.63 | 15.57 |
| 5 | 5.355 | 9.64 | 27.1 |
| 6 | 3.9 | 4.56 | 16.44 |
| 7 | 0.26 | 0.49 | 6.89^{*} |
| 8 | 58.41 | 5.5 | 11.59 |
| 9 | 20.82 | 4.85 | 7.69 |
| 10 | 6.1 | 0.109 | 1.2 |
| 11 | 8.85 | 1.63 | 2.47 |
| 12 | 33.49 | 32.52 | 172.3 |
| 13 | 5.16 | 35.59 | 10.35 |
| 14 | 2.19 | 0.59 | 3.19 |
| 15 | 3.23 | 0.258 | 3.46 |
| 16 | 1.75 | 0.169 | 5.22 |
| 17 | 6.3 | 67.14 | 111.29 |
| 18 | 317.95 | >3000 | 188.99 * |
| 19 | 110.73 | 20.88 | 21.64 |
| 20 | 0.05 | 0.126 | 0.91 |
| 21 | 0.376 | 0.053 | 3.51 |
| 22 | 14.16 | 0.055 | 2.75 |
| 23 | 13.58 | 0.55 | 1.47 |
| 24 | 30.32 | >3000 | 5.99 |
| 25 | 172.85 | 5.69 | 17.44 |
| 26 | 34.57 | 0.88 | 3.13 |
| 27 | 146.84 | 68.28 | >1000 |
| 28 | 48.9 | 3.53 | 5.86 |
| 29 | 20.95 | 1.42 | 4.27 |
| 30 | 31.55 | 10.15 | 4.05 |
| 31 | 140.68 | 15.22 | 17.5 |
| 32 | 3.55 | 0.634 | 9.89 |
| 33 | 0.175 | 0.34 | 0.021 |
| 34 | 560.13 | | |
| 35 | 3.49 | 0.265 | 7.09 |
| 36 | 4.37 | 0.052 | 2.52 |
| 37 | 2.86 | 0.143 | 3.07 |
| 38 | 2.34 | 0.956 | 9.44 |
| 39 | 4.92 | 0.926 | 2.31 |
| 40 | 2720.46 | | * |
| 41 | 88.01 | 575.43 | >3000 * |
| 42 | 118.2 | 782.18 | >10000 * |
| 43 | 39.9 | 3.68 | 2.34 |
| 44 | 3.93 | 0.208 | 7.4 |
| 45 | 4.013 | 0.005 | 0.016 |
| 46 | 60.56 | 490.14 | 32.54 |
| 47 | 1076.76 | | |
| 48 | 470.84 | >1000 | >1000 |
| 49 | 51.12 | 40.13 | 119.03 |
| 50 | 80.15 | 11.31 | 94.24 |
| 51 | 36.81 | 3.26 | 32.92 |
| 52 | 94.41 | 18.33 | 107.17 |
| 53 | 64.15 | 14.25 | 40.82 |
| 54 | 40.79 | 3.19 | 19.56 |
| 55 | 32.82 | 5.84 | 19.86 |
| 56 | 25.72 | 6.81 | 25.76 |
| 57 | 34.02 | 15.93 | 39.29 |
| 58 | 30.65 | 11.65 | 60.99 |
| 59 | 40.79 | 7.94 | 34.11 |
| 60 | 34.29 | | |
| 61 | 29.83 | | |
| 62 | 58.39 | | |
| 63 | 0.59 | 0.0002 | 0.18 |
| 64 | 13.09 | 0.138 | 4.61 |
| 65 | 574.71 | 244.96 | 163.36 |
| 66 | 4.21 | 0.069 | 15.59 |
| 67 | 13.4 | 0.618 | 4.37 |
| 68 | 7.59 | 0.73 | 34.84 |
| 69 | 2261 | 90.16 | >1000 |
| 70 | 9.89 | 0.44 | 20.13 |
| 71 | 17.24 | 3.39 | 2.42 |
| 72 | 12.64 | 2.54 | 6.24 |
| 73 | 4.925 | 0.06 | 9.7 |
| 74 | 14.67 | 5.7 | 7.28 |
| 75 | 23.72 | 1.51 | 78.33 |
| 76 | 33.03 | 22.13 | >500 |
| 77 | 6.254 | 0.68 | >500 |
| 78 | 17.65 | 1.58 | >500 |
| 79 | 8.03 | 12.48 | >1000 |
| 80 | 69.08 | 15.86 | 55.99 |
| 81 | 228.7 | 29.03 | 33.63 |
| 82 | 20.24 | 1.36 | 29.58 |
| 83 | 200.06 | 74.87 | 117.05 |
| 84 | 173.98 | 24.71 | 27.42 |
| 85 | 507.72 | | |
| 86 | 244.07 | >1000 | 39.26 |
| 87 | 98.93 | 39.45 | >300 |
| 88 | 129.6 | 48.87 | >300 |
| 89 | 5.85 | 1.12 | 11.16 |
| 90 | 202.17 | 57.7 | >300 |
| 91 | 208.32 | 22.07 | 14.67 |
| 92 | 38.82 | 13.9 | 32.88 |
| 93 | 64.05 | 23.57 | 104.31 |
| 94 | 49.55 | >1000 | 35.99 |
| 95 | 338.13 | >1000 | 110.22 |
| 96 | 48.55 | 10.08 | 52.45 |

| | | | |
|---|---|---|---|
| ^{*} COMPARATIVE EXAMPLE | | | |

## Claims

1. A compound having the structure of Formula I 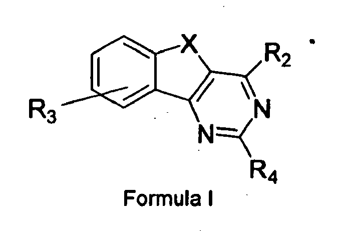 or a pharmaceutically acceptable salt thereof, wherein
(b) R₂ is selected from the group consisting of optionally substituted aryl, and optionally substituted heteroaryl;
(c) R₃ is from one to four groups independently selected from the group consisting of:
hydrogen, halo, C₁₋₈ straight or branched chain alkyl, arylalkyl, C₃₋₇ cycloalkyl, C₁₋₈ alkoxy, cyano, C₁₋₄ carboalkoxy, trifluoromethyl, C₁₋₈ alkylsulfonyl, halogen, nitro, hydroxy, trifluoromethoxy, C₁₋₈ carboxylate, aryl, heteroaryl, and heterocyclyl, -NR₁₁R₁₂,
wherein R₁₁ and R₁₂ are independently selected from H, C₁₋₈ straight or branched chain alkyl, arylalkyl, C₃₋₇ cycloalkyl, carboxyalkyl, aryl, heteroaryl, and heterocyclyl or R₁₀ and R₁₁ taken together with the nitrogen form a heteroaryl or heterocyclyl group,
-NR₁₃COR₁₄,
wherein R₁₃ is selected from hydrogen or alkyl and R₁₄ is selected from hydrogen, alkyl, substituted alkyl, C₁₋₃ alkoxyl, carboxyalkyl, aryl, arylalkyl, heteroaryl, heterocyclyl, R₁₅R₁₆N
(CH₂)ₚ-, or R₁₅R₁₆NCO(CH₂)ₚ-, wherein R₁₅ and R₁₆ are independently selected from H, OH, alkyl, and alkoxy, and p is an integer from 1-6,
wherein the alkyl group may be substituted with carboxyl, alkyl, aryl, substituted aryl, heterocyclyl, substituted heterocyclyl, heteroaryl, substituted heteroaryl, hydroxamic acid, sulfonamide, sulfonyl, hydroxy, thiol, alkoxy or arylalkyl, or R₁₃ and R₁₄ taken together with the carbonyl form a carbonyl containing heterocyclyl group;
(d) R₄ is selected from the group consisting of hydrogen, C₁₋₆ straight or branched chain alkyl, benzyl
wherein the alkyl and benzyl groups are optionally substituted with one or more groups selected from C₃₋₇ cycloalkyl, C₁₋₈ alkoxy, cyano, C₁₋₄ carboalkoxy, trifluoromethyl, C₁₋₈ alkylsulfonyl, halogen, nitro, hydroxy, trifluoromethoxy, C₁₋₈ carboxylate, amino, NR₁₇R₁₈, aryl and heteroaryl,
-OR₁₇, and -NR₁₇R₁₈,
wherein R₁₇ and R₁₈ are independently selected from hydrogen, and optionally substituted C₁₋₆ alkyl or aryl; and
(e) X is selected from C=S, C=O; CH₂, CHOH, CHOR_{19,} or CHNR₂₀R₂₁ where R_{19,} R_{20,} and R₂₁ are selected from optionally substituted C₁₋₈ straight of branched chain alkyl, wherein the substituents on the alkyl group are selected from C₁₋₈ alkoxy, hydroxy, halogen, amino, cyano, or NR₂₂R₂₃ wherein R₂₂ and R₂₃ are independently selected from the group consisting of hydrogen, C₁₋₈ straight or branched chain alkyl, C₃₋₇ cycloalkyl, benzyl, aryl, heteroaryl, or NR₂₂R₂₃ taken together from a heterocycle or heteroaryl;
wherein, unless otherwise stated,
"alkyl" means straight, cyclic or branched-chain alkyl containing 1-20 carbon atoms and optionally substituted with one or more groups such as halogen, OH, CN, mercapto, nitro, amino, C₁-C₈-alkyl, C₁-C₈-alkoxyl, C₁-C₈-alkylthio, C₁-C₈-alkyl-amino, di(C₁-C₈-alkyl)amino, (mono-, di-, tri-, and per-) halo-alkyl, formyl, carboxy, alkoxycarbonyl, C₁-C₈-alkyl-CO-O-, C₁-C₈-alkyl-CO-NH-, carboxamide, hydroxamic acid, sulfonamide, sulfonyl, thiol, aryl, aryl(C₁-C₈)alkyl, heterocyclyl and heteroaryl;
"aryl" or "Ar," whether used alone or as part of a substituent group, is a carbocyclic aromatic radical that may be substituted by independent replacement of 1 to 5 of the hydrogen atoms thereon with halogen, OH, CN, mercapto, nitro, amino, C₁-C₈-alkyl, C₁-C₈-alkoxyl, C₁-C₈-alkylthio, C₁-C₈-alkyl-amino, di(C₁-C₈-alkyl)amino, (mono-, di-,tri-, and per-) halo-alkyl, formyl, carboxy, alkoxycarbonyl, C₁-C₈-alkyl-CO-O-, C₁-C₈-alkyl-CO-NH-, or carboxamide; and
"heterocycle", "heterocyclic" and "heterocyclo" refer to an optionally substituted, fully or partially saturated cyclic group which is, for example, a 4- to 7-membered monocyclic, 7-to 11-membered bicyclic, or 10- to 15-membered tricyclic ring system, which has at least one heteroatom in at least one carbon atom containing ring and wherein each ring of the heterocyclic group containing a heteroatom may have 1, 2, or 3 heteroatoms selected from nitrogen atoms, oxygen atoms, and sulfur atoms, where the nitrogen and sulfur heteroatoms may also optionally be oxidized and the nitrogen atoms may optionally be quaternized;
with the proviso that said compound is not: 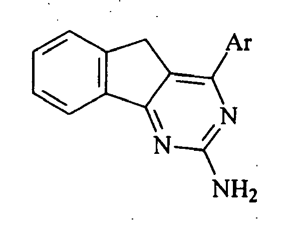 wherein Ar is phenyl, *p*-methoxyphenyl, *m*-methoxyphenyl, *p*-bromophenyl, *m*-bromophenyl, *p*-chlorophenyl, *o*-chlorophenyl, 1-naphthyl, 2-naphthyl, 2-thienyl or 2-furanyl; or 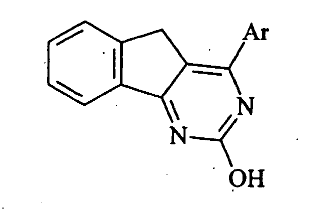 wherein Ar is p-chlorophenyl, 1-naphthyl, or 2-thienyl.

2. A compound having the structure of Formula II 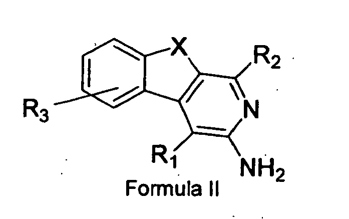 or a pharmaceutically acceptable salt thereof, wherein
(a) R₁ is selected from the group consisting of
(i) -COR₅, wherein R₅ is selected from H, optionally substituted C₁₋₈ straight or branched chain alkyl, optionally substituted aryl and optionally substituted arylalkyl;
wherein the substituents on the alkyl, aryl and arylalkyl group are selected from C₁₋₈ alkoxy, phenylacetyloxy, hydroxy, halogen, p-tosyloxy, mesyloxy, amino, cyano, carboalkoxy, or NR₇R₈
wherein R₇ and R₈ are independently selected from the group consisting of hydrogen, C₁₋₈ straight or branched chain alkyl, C₃₋₇ cycloalkyl, benzyl, aryl, or heteroaryl or NR₇R₈ taken together form a heterocycle or heteroaryl;
(ii) COOR₅, wherein R₅ is as defined above;
(iii) cyano;
(iv) -CONR₉R₁₀ wherein R₉ and R₁₀ are independently selected from H, C₁₋₈ straight or branched chain alkyl, C₃₋₇ cycloalkyl, trifluoromethyl, hydroxy, alkoxy, acyl, alkylcarbonyl, carboxyl, arylalkyl, aryl, heteroaryl and heterocyclyl;
wherein the alkyl, cycloalkyl, alkoxy, acyl, alkylcarbonyl, carboxyl, arylalkyl, aryl, heteroaryl and heterocyclyl groups may be substituted with carboxyl, alkyl, aryl, substituted aryl, heterocyclyl, substituted heterocyclyl, heteroaryl, substituted heteroaryl, hydroxamic acid, sulfonamide, sulfonyl, hydroxy, thiol, amino, alkoxy or arylalkyl, or R₉ and R₁₀ taken together with the nitrogen to which they are attached form a heterocycle or heteroaryl group;
(v) optionally substituted C₁₋₈ straight or branched chain alkyl; wherein the substituents on the alkyl, group are selected from C₁₋₈ alkoxy, phenylacetyloxy, hydroxy, halogen, p-tosyloxy, mesyloxy, amino, cyano, carboalkoxy, carboxyl, aryl, heterocyclyl, heteroaryl, sulfonyl, thiol, alkylthio, or NR₇R₈ wherein R₇ and R₈ are as defined above;
(b) R₂ is selected from the group consisting of optionally substituted alkyl, optionally substituted aryl, optionally substituted heteroaryl, optionally substituted heterocyclyl and optionally substituted C₃₋₇ cycloalkyl, C₁₋₈ alkoxy, aryloxy, C₁₋₈ alkylsulfonyl, arylsulfonyl, arylthio, C₁₋₈ alkylthio, or -NR₂₄R₂₅
wherein R₂₄ and R₂₅ are independently selected from H, C₁₋₈ straight or branched chain alkyl, arylalkyl, C₃₋₇ cycloalkyl, carboxyalkyl, aryl, heteroaryl, and heterocyclyl or R₂₄ and R₂₅ taken together with the nitrogen form a heteroaryl or heterocyclyl group,
(c) R₃ is from one to four groups independently selected from the group consisting of:
hydrogen, halo, C₁₋₈ straight or branched chain alkyl, arylalkyl, C₃₋₇ cycloalkyl, C₁₋₈ alkoxy, cyano, C₁₋₄ carboalkoxy, trifluoromethyl, C₁₋₈ alkylsulfonyl, halogen, nitro, hydroxy, trifluoromethoxy, C₁₋₈ carboxylate, aryl, heteroaryl, and heterocyclyl, -NR₁₁R₁₂,
wherein R₁₁ and R₁₂ are independently selected from H, C₁₋₈ straight or branched chain alkyl, arylalkyl, C₃₋₇ cycloalkyl, carboxyalkyl, aryl, heteroaryl, and heterocyclyl or R₁₀ and R₁₁ taken together with the nitrogen form a heteroaryl or heterocyclyl group,
-NR₁₃COR₁₄,
wherein R₁₃ is selected from hydrogen or alkyl and R₁₄ is selected from hydrogen, alkyl, substituted alkyl, C₁₋₃ alkoxyl, carboxyalkyl, aryl, arylalkyl, heteroaryl, heterocyclyl, R₁₅R₁₆N
(CH₂)ₚ-, or R₁₅R₁₆NCO(CH₂)ₚ-, wherein R₁₅ and R₁₆ are independently selected from H, OH, alkyl, and alkoxy, and p is an integer from 1-6,
wherein the alkyl group may be substituted with carboxyl, alkyl, aryl, substituted aryl, heterocyclyl, substituted heterocyclyl, heteroaryl, substituted heteroaryl, hydroxamic acid, sulfonamide, sulfonyl, hydroxy, thiol, alkoxy or arylalkyl, or R₁₃ and R₁₄ taken together with the carbonyl form a carbonyl containing
heterocyclyl group;
(e) X is selected from C=S, C=O; CH₂, CHOH, CHOR_{19;} or CHNR₂₀R₂₁ where R₁₉, R₂₀, and R₂₁ are selected from optionally substituted C₁₋₈ straight of branched chain alkyl, wherein the substituents on the alkyl group are selected from C₁₋₈ alkoxy, hydroxy, halogen, amino, cyano, or NR₂₂R₂₃ wherein R₂₂ and R₂₃ are independently selected from the group consisting of hydrogen, C₁₋₈ straight or branched chain alkyl, C₃₋₇ cycloalkyl, benzyl, aryl, heteroaryl, or NR₂₂R₂₃ taken together from a heterocycle or heteroaryl;
wherein, unless otherwise stated,
"alkyl" means straight, cyclic or branched-chain alkyl containing 1-20 carbon atoms and optionally substituted with one or more groups such as halogen, OH, CN, mercapto, nitro, amino, C₁-C₈-alkyl, C₁-C₈-alkoxyl, C₁-C₈-alkylthio, C₁-C₈-alkyl-amino, di(C₁-C₈-alkyl)amino, (mono-, di-, tri-, and per-) halo-alkyl, formyl, carboxy, alkoxycarbonyl, C₁-C₈-alkyl-CO-O-, C₁-C₈-alkyl-CO-NH-, carboxamide, hydroxamic acid, sulfonamide, sulfonyl, thiol, aryl, aryl(C₁-C₈)alkyl, heterocyclyl and heteroaryl;
"aryl" or "Ar," whether used alone or as part of a substituent group, is a carbocyclic aromatic radical that may be substituted by independent replacement of 1 to 5 of the hydrogen atoms thereon with halogen, OH, CN, mercapto, nitro, amino, C₁-C₈-alkyl, C₁-C₈-alkoxyl, C₁-C₈-alkylthio, C₁-C₈-alkyl-amino, di(C₁-C₈-alkyl)amino, (mono-, di-,tri-, and per-) halo-alkyl, formyl, carboxy, alkoxycarbonyl, C₁-C₈-alkyl-CO-O-, C₁-C₈-alkyl-CO-NH-, or carboxamide; and
"heterocycle", "heterocyclic" and "heterocyclo" refer to an optionally substituted, fully or partially saturated cyclic group which is, for example, a 4- to 7-membered monocyclic, 7-to 11-membered bicyclic, or 10- to 15-membered tricyclic ring system, which has at least one heteroatom in at least one carbon atom containing ring and wherein each ring of the heterocyclic group containing a heteroatom may have 1, 2, or 3 heteroatoms selected from nitrogen atoms, oxygen atoms, and sulfur atoms, where the nitrogen and sulfur heteroatoms may also optionally be oxidized and the nitrogen atoms may optionally be quaternized;
with the proviso that when R₁ is a cyano, then R₂ is not phenyl.

3. The compound of claim 2, wherein R₂ is optionally substituted heteroaryl or optionally substituted aryl.

4. The compound of claim 1 or claim 3, wherein R₂ is an optionally substituted furan.

5. The compound of any one of the preceding claims, wherein R₁ is COOR₅ and R₅ is an optionally substituted C₁₋₈ straight or branched chain alkyl.

6. The compound of any one of the preceding claims, wherein X is C=O:

7. The compound of any one of claims 1 and 4-6, wherein the compound is of Formula I and R₄ is amino.

8. The compound of Claim 1 or 2, which is:
2-amino-4-furan-2-yl-indeno[1,2-d]pyrimidin-5-one;
2-amino-4-phenyl-indeno[1,2-d]pyrimidin-5-one;
2-amino-4-thiophen-2-yl-indeno[1,2-d]pyrimidin-5-one;
2-amino-4-(5-methyl-furan-2-yl)-indeno[1,2-d]pyrimidin-5-one;
2,6-diamino-4-furan-2-yl-indeno[1,2-d]pyrimidin-5-one;
9*H*-indeno[2,1-c]pyridine-4-carbonitrile, 3-amino-1-furan-2-yl-9-oxo-:
9*H*-indeno[2,1-c]pyridine-4-carboxylic acid, 3-amino-1-furan-2-yl-9-oxo-, 2-dimethylamino-ethyl ester;
9*H*-indeno[2,1-c]pyridine-4-carboxylic acid, 3-amino-1-phenyl-9-oxo-, 2-dimethylamino-ethyl ester;
9*H*-indeno[2,1-c]pyridine-4-carboxylicacid, 3-aminol-furan-2-yl-9-oxo-, (2-dimethylamino-1-methyl-ethyl)-amide;
9*H*-indeno[2,1-c]pyridine-4-carboxylic acid, 3-amino-1-furan-2-yl-9-oxo-, (2-dimethylamino-ethyl)-methyl-amide; or
9*H*-indeno[2,1-c]pyridine-4-carboxylic acid, 3-amino-1-furan-2-yl-9-oxo-, 1-methyl-pyrrolidin-2-ylmethyl ester.

9. A pharmaceutical composition comprising the compound of any one of claims 1 to 8 and a pharmaceutically acceptable carrier, wherein the proviso of claim 1 does not apply.

10. A compound of any one of claims 1 to 8 for use in medicine, wherein the proviso of claim 1 does not apply.

11. A compound of any one of claims 1 to 8 or a pharmaceutical composition of claim 8 for use in treating a subject having, or preventing in a subject, a disorder ameliorated by antagonizing Adenosine A2a receptors in appropriate cells, such as a neurodegenerative disorder or a movement disorder, for instance Parkinson's Disease, Huntington's Disease, Multiple System Atrophy, Corticobasal Degeneration, Alzheimer's Disease, and Senile Dementia, wherein the proviso of claim 1 does not apply.

## Patentansprüche

1. Verbindung mit der Struktur von Formel I 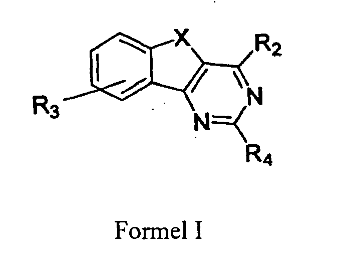 oder ein pharmazeutisch verträgliches Salz davon, wobei
(b) R₂ ausgewählt ist aus der Gruppe, bestehend aus gegebenenfalls substituiertem Aryl und gegebenenfalls substituiertem Heteroaryl;
(c) R₃ von einer bis vier Gruppen ist, die unabhängig ausgewählt ist/sind aus der Gruppe, bestehend aus:
Wasserstoff, Halo, gerad- oder verzweigtkettigem C₁₋₈-Alkyl, Arylalkyl, C₃₋₇-Cycloalkyl, C₁₋₈-Alkoxy, Cyano, C₁₋₄-Carboalkoxy, Trifluormethyl, C₁₋₈-Alkylsulfonyl, Halogen, Nitro, Hydroxy, Trifluormethoxy, C₁₋₈-Carboxylat, Aryl, Heteroaryl und Heterocyclyl, -NR₁₁R₁₂,
wobei R₁₁ und R₁₂ unabhängig ausgewählt sind aus H, gerad- oder verzweigtkettigem C₁₋₈-Alkyl, Arylalkyl, C₃₋₇-Cycloalkyl, Carboxyalkyl, Aryl, Heteroaryl und Heterocyclyl oder R₁₀ und R₁₁ zusammengenommen mit dem Stickstoff eine Heteroaryl- oder Heterocyclylgruppe bilden,
- NR₁₃COR₁₄,
wobei R₁₃ ausgewählt ist aus Wasserstoff oder Alkyl und R₁₄ ausgewählt ist aus Wasserstoff, Alkyl, substituiertem Alkyl, C₁₋₃-Alkoxyl, Carboxyalkyl, Aryl, Arylalkyl, Heteroaryl, Heterocyclyl, R₁₅R₁₆N(CH₂)ₚ- oder R₁₅R₁₆NCO(CH₂)ₚ-, wobei R₁₅ und R₁₆ unabhängig ausgewählt sind aus H, OH, Alkyl und Alkoxy und p eine ganze Zahl von 1-6 ist,
wobei die Alkylgruppe mit Carboxyl, Alkyl, Aryl, substituiertem Aryl, Heterocyclyl, substituiertem Heterocyclyl, Heteroaryl, substituiertem Heteroaryl, Hydroxamsäure, Sulfonamid, Sulfonyl, Hydroxy, Thiol, Alkoxy oder Arylalkyl substituiert sein kann oder R₁₃ und R₁₄ zusammengenommen mit dem Carbonyl eine carbonylhaltige Heterocyclylgruppe bilden;
(d) R₄ ausgewählt ist aus der Gruppe, bestehend aus Wasserstoff, gerad- oder verzweigtkettigem C₁₋₆-Alkyl, Benzyl,
wobei die Alkyl- und Benzylgruppen gegebenenfalls mit einer oder mehreren Gruppen substituiert sind, die ausgewählt ist/sind aus C₃₋₇-Cycloalkyl, C₁₋₈-Alkoxy, Cyano, C₁₋₄-Carboalkoxy, Trifluormethyl, C₁₋₈ -Alkylsulfonyl, Halogen, Nitro, Hydroxy, Trifluormethoxy, C₁₋₈-Carboxylat, Amino, NR₁₇R₁₈, Aryl und Heteroaryl,
-OR₁₇ und -NR₁₇R₁₈,
wobei R₁₇ und R₁₉ unabhängig ausgewählt sind aus Wasserstoff und gegebenenfalls substituiertem C₁₋₆-Alkyl oder Aryl; und
(e) X ausgewählt ist aus C=S, C=O; CH₂, CHOH, CHOR₁₉ oder CHNR₂₀R₂₁, wobei R₁₉, R₂₀ und R₂₁ ausgewählt sind aus gegebenenfalls substituiertem, gerad- oder verzweigtkettigem C₁₋₈-Alkyl, wobei die Substituenten auf der Alkylgruppe ausgewählt sind aus C₁₋₈-Alkoxy, Hydroxy, Halogen, Amino, Cyano oder NR₂₂R₂₃, wobei R₂₂ und R₂₃ unabhängig ausgewählt sind aus der Gruppe, bestehend aus Wasserstoff, gerad- oder verzweigtkettigem C₁₋₈-Alkyl, C₃₋₇-Cycloalkyl, Benzyl, Aryl, Heteroaryl, oder NR₂₂R₂₃ zusammengenommen einen Heterocyclus oder Heteroaryl bilden;
wobei, sofern nicht anders angegeben,
"Alkyl" geradkettiges, cyclisches oder verzweigtkettiges Alkyl bedeutet, das 1-20 Kohlenstoffatome enthält und gegebenenfalls mit einer oder mehreren Gruppen substituiert ist, wie etwa Halogen, OH, CN, Mercapto, Nitro, Amino, C₁-C₈-Alkyl, C₁-C₈-Alkoxyl, C₁-C₈-Alkylthio, C₁-C₈-Alkylamino, Di(C₁₋-C₈-alkyl)amino, (Mono-, Di-, Tri- und Per-)haloalkyl, Formyl, Carboxy, Alkoxycarbonyl, C₁-C₈-Alkyl-CO-O-, C₁-C₈-Alkyl-CO-NH-, Carboxamid, Hydroxamsäure, Sulfonamid, Sulfonyl, Thiol, Aryl, Aryl(C₁-C₈)alkyl, Heterocyclyl und Heteroaryl;
"Aryl" oder "Ar", ob allein oder als Teil einer Substituentengruppe verwendet, ein carbocyclischer aromatischer Rest ist, der durch unabhängigen Ersatz von 1 bis 5 der Wasserstoffatome daran mit Halogen, OH, CN, Mercapto, Nitro, Amino, C₁-C₈-Alkyl, C₁-C₈-Alkoxyl, C₁-C₈-Alkylthio, C₁-C₈-Alkylamino, Di(C₁-C₈-alkyl)amino, (Mono-, Di-, Tri- und Per-)haloalkyl, Formyl, Carboxy, Alkoxycarbonyl, C₁-C₈-Alkyl-CO-O-, C₁-C₈-Alkyl-CO-NH- oder Carboxamid substituiert sein kann; und
"Heterocyclus", "heterocyclisch" und "Heterocyclo" sich auf eine gegebenenfalls substituierte, vollständig oder teilweise gesättigte cyclische Gruppe beziehen, die zum Beispiel ein 4- bis 7-gliedriges monocyclisches, 7-bis 11-gliedriges bicyclisches oder 10- bis 15-gliedriges tricyclisches Ringsystem ist, die wenigstens ein Heteroatom in wenigstens einem kohlenstoffatomhaltigen Ring aufweist und wobei jeder Ring der heterocyclischen Gruppe, der ein Heteroatom enthält, 1, 2 oder 3 Heteroatome aufweisen kann, die ausgewählt sind aus Stickstoffatomen, Sauerstoffatomen und Schwefelatomen, wobei die Stickstoff- und Schwefel-Heteroatome gegebenenfalls auch oxidiert sein können und die Stickstoffatome gegebenenfalls quatemisiert sein können;
mit der Maßgabe, dass die Verbindung nicht: 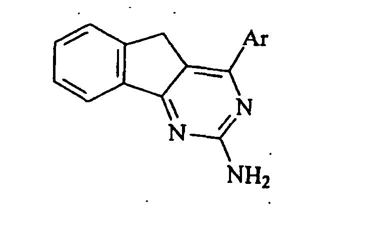 wobei Ar Phenyl, p-Methoxyphenyl, m-Methoxyphenyl, p-Bromphenyl, m-Bromphenyl, p-Chlorphenyl, o-Chlorphenyl, 1-Naphthyl, 2-Naphthyl, 2-Thienyl oder 2-Furanyl ist; oder 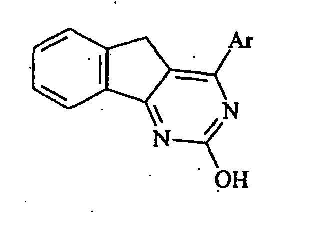 wobei Ar p-Chlorphenyl, 1-Naphthyl oder 2-Thienyl ist, ist.

2. Verbindung mit der Struktur von Formel II 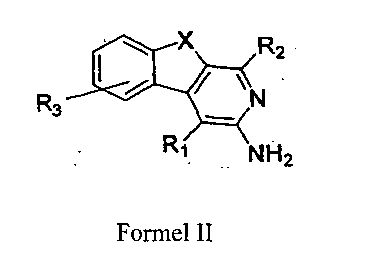 oder ein pharmazeutisch verträgliches Salz davon, wobei
(a) R₁ ausgewählt ist aus der Gruppe, bestehend aus
(i) -COR₅, wobei R₅ ausgewählt ist aus H, gegebenenfalls substituiertem, gerad- oder verzweigtkettigem C₁₋₈-Alkyl, gegebenenfalls substituiertem Aryl und gegebenenfalls substituiertem Arylalkyl; wobei die Substituenten auf der Alkyl-, Aryl- und Aralkylgruppe ausgewählt sind aus C₁₋₈-Alkoxy, Phenylacetyloxy, Hydroxy, Halogen, p-Tosyloxy, Mesyloxy, Amino, Cyano, Carboalkoxy oder NR₇R₈, wobei R₇ und R₈ unabhängig ausgewählt sind aus der Gruppe, bestehend aus Wasserstoff, gerad- oder verzweigtkettigem C₁₋₈-Alkyl, C₃₋₇-Cycloalkyl, Benzyl, Aryl oder Heteroaryl, oder NR₇R₈ zusammengenommen einen Heterocyclus oder Heteroaryl bilden;
(ii) COOR₅, wobei R₅ ist, wie oben definiert;
(iii) Cyano;
(iv) -CONR₉R₁₀, wobei R₉ und R₁₀ unabhängig ausgewählt sind aus H, gerad- oder verzweigtkettigem C₁₋₈-Alkyl, C₃₋₇-Cycloalkyl, Trifluormethyl, Hydroxy, Alkoxy, Acyl, Alkylcarbonyl, Carboxyl, Arylalkyl, Aryl, Heteroaryl und Heterocyclyl;
wobei die Alkyl-, Cycloalkyl-, Alkoxy-, Acyl-, Alkylcarbonyl-, Carboxyl-, Arylalkyl-, Aryl-, Heteroaryl- und Heterocyclylgruppen mit Carboxyl, Alkyl, Aryl, substituiertem Aryl, Heterocyclyl, substituiertem Heterocyclyl, Heteroaryl, substituiertem Heteroaryl, Hydroxamsäure, Sulfonamid, Sulfonyl, Hydroxy, Thiol, Amino, Alkoxy oder Arylalkyl substituiert sein können oder R₉ und R₁₀ zusammengenommen mit dem Stickstoff, an das sie gebunden sind, eine Heterocyclus- oder Heteroarylgruppe bilden;
(v) gegebenenfalls substituiertem, gerad- oder verzweigtkettigem C₁₋₈-Alkyl;
wobei die Substituenten auf der Alkylgruppe ausgewählt sind aus C₁₋₈-Alkoxy, Phenylacetyloxy, Hydroxy, Halogen, p-Tosyloxy, Mesyloxy, Amino, Cyano, Carboalkoxy, Carboxyl, Aryl, Heterocyclyl, Heteroaryl, Sulfonyl, Thiol, Alkylthiol oder NR₇R₈, wobei R₇ und R₈ sind, wie oben definiert;
(b) R₂ ausgewählt ist aus der Gruppe, bestehend aus gegebenenfalls substituiertem Alkyl, gegebenenfalls substituiertem Aryl, gegebenenfalls substituiertem Heteroaryl, gegebenenfalls substituiertem Heterocyclyl und gegebenenfalls substituiertem C₃₋₇-Cycloalkyl, C₁₋₈-Alkoxy, Aryloxy, C₁₋₈-Alkylsulfonyl, Arylsulfonyl, Arylthio, C₁₋₈-Alkylthio oder -NR₂₄R₂₅,
wobei R₂₄ und R₂₅ unabhängig ausgewählt sind aus H, gerad- oder verzweigtkettigem C₁₋₈-Alkyl, Arylalkyl, C₃₋₇-Cycloalkyl, Carboxyalkyl, Aryl, Heteroaryl und Heterocyclyl oder R₂₄ und R₂₅ zusammengenommen mit dem Stickstoff eine Heteroaryl- oder Heterocyclylgruppe bilden,
(c) R₃ von einer bis vier Gruppen ist, die unabhängig ausgewählt ist/sind aus der Gruppe, bestehend aus:
Wasserstoff, Halo, gerad- oder verzweigtkettigem C₁₋₈-Alkyl, Arylalkyl, C₃₋₇-Cycloalkyl, C₁₋₈-Alkoxy, Cyano, C₁₋₄-Carboalkoxy, Trifluormethyl, C₁₋₈-Alkylsulfonyl, Halogen, Nitro, Hydroxy, Trifluormethoxy, C₁₋₈-Carboxylat, Aryl, Heteroaryl und Heterocyclyl, -NR₁₁R₁₂,
wobei R₁₁ und R₁₂ unabhängig ausgewählt sind aus H, gerad- oder verzweigtkettigem C₁₋₈-Alkyl, Arylalkyl, C₃₋₇-Cycloalkyl, Carboxyalkyl, Aryl, Heteroaryl und Heterocyclyl oder R₁₀ und R₁₁ zusammengenommen mit dem Stickstoff eine Heteroaryl- oder Heterocyclylgruppe bilden,
-NR₁₃COR₁₄,
wobei R₁₃ ausgewählt ist aus Wasserstoff oder Alkyl und R₁₄ ausgewählt ist aus Wasserstoff, Alkyl, substituiertem Alkyl, C₁₋₃-Alkoxyl, Carboxyalkyl, Aryl, Arylalkyl, Heteroaryl, Heterocyclyl, R₁₅R₁₆N(CH₂)ₚ- oder R₁₅R₁₆NCO(CH₂)ₚ-, wobei R₁₅ und R₁₆ unabhängig ausgewählt sind aus H, OH, Alkyl und Alkoxy und p eine ganze Zahl von 1-6 ist,
wobei die Alkylgruppe mit Carboxyl, Alkyl, Aryl, substituiertem Aryl, Heterocyclyl, substituiertem Heterocyclyl, Heteroaryl, substituiertem Heteroaryl, Hydroxamsäure, Sulfonamid, Sulfonyl, Hydroxy, Thiol, Alkoxy oder Arylalkyl substituiert sein kann oder R₁₃ und R₁₄ zusammengenommen mit dem Carbonyl eine carbonylhaltige Heterocyclylgruppe bilden;
(d) X ausgewählt ist aus C=S, C=O; CH₂, CHOH, CHOR₁₉ oder CHNR₂₀R₂₁, wobei R₁₉, R₂₀ und R₂₁ ausgewählt sind aus gegebenenfalls substituiertem, gerad- oder verzweigtkettigem C₁₋₈-Alkyl, wobei die Substituenten auf der Alkylgruppe ausgewählt sind aus C₁₋₈-Alkoxy, Hydroxy, Halogen, Amino, Cyano oder NR₂₂R₂₃, wobei R₂₂ und R₂₃ unabhängig ausgewählt sind aus der Gruppe, bestehend aus Wasserstoff, gerad- oder verzweigtkettigem C₁₋₈-Alkyl, C₃₋₇-Cycloalkyl, Benzyl, Aryl, Heteroaryl, oder NR₂₂R₂₃ zusammengenommen einen Heterocyclus oder Heteroaryl bilden;
wobei, sofern nicht anders angegeben,
"Alkyl" geradkettiges, cyclisches oder verzweigtkettiges Alkyl bedeutet, das 1-20 Kohlenstoffatome enthält und gegebenenfalls mit einer oder mehreren Gruppen substituiert ist, wie etwa Halogen, OH, CN, Mercapto, Nitro, Amino, C₁-C₈-Alkyl, C₁-C₈-Alkoxyl, C₁-C₈-Alkylthio, C₁-C₈-Alkylamino, Di(C₁₋-C₈-alkyl)amino, (Mono-, Di-, Tri- und Per-)haloalkyl, Formyl, Carboxy, Alkoxycarbonyl, C₁-C₈-Alkyl-CO-O-, C₁-C₈-Alkyl-CO-NH-, Carboxamid, Hydroxamsäure, Sulfonamid, Sulfonyl, Thiol, Aryl, Aryl(C₁-C₈)alkyl, Heterocyclyl und Heteroaryl;
"Aryl" oder "Ar", ob allein oder als Teil einer Substituentengruppe verwendet, ein carbocyclischer aromatischer Rest ist, der durch unabhängigen Ersatz von 1 bis 5 der Wasserstoffatome daran mit Halogen, OH, CN, Mercapto, Nitro, Amino, C₁-C₈-Alkyl, C₁-C₈-Alkoxyl, C₁-C₈-Alkylthio, C₁-C₈-Alkylamino, Di(C₁-C₈-alkyl)amino, (Mono-, Di-, Tri- und Per-)haloalkyl, Formyl, Carboxy, Alkoxycarbonyl, C₁-C₈-Alkyl-CO-O-, C₁-C₈-Alkyl-CO-NH- oder Carboxamid substituiert sein kann; und
"Heterocyclus", "heterocyclisch" und "Heterocyclo" sich auf eine gegebenenfalls substituierte, vollständig oder teilweise gesättigte cyclische Gruppe beziehen, die zum Beispiel ein 4- bis 7-gliedriges monocyclisches, 7-bis 11-gliedriges bicyclisches oder 10- bis 15-gliedriges tricyclisches Ringsystem ist, die wenigstens ein Heteroatom in wenigstens einem kohlenstoffatomhaltigen Ring aufweist und wobei jeder Ring der heterocyclischen Gruppe, der ein Heteroatom enthält, 1, 2 oder 3 Heteroatome aufweisen kann, die ausgewählt sind aus Stickstoffatomen, Sauerstoffatomen und Schwefelatomen, wobei die Stickstoff- und Schwefel-Heteroatome gegebenenfalls auch oxidiert sein können und die Stickstoffatome gegebenenfalls quatemisiert sein können;
mit der Maßgabe, dass, wenn R₁ Cyano ist, dann R₂ nicht Phenyl ist.

3. Verbindung nach Anspruch 2, wobei R₂ gegebenenfalls substituiertes Heteroaryl oder gegebenenfalls substituiertes Aryl ist.

4. Verbindung nach Anspruch 1 oder Anspruch 3, wobei R₂ gegebenenfalls substituiertes Furan ist.

5. Verbindung nach einem der vorangehenden Ansprüche, wobei R₁ COOR₅ ist und R₅ ein gegebenenfalls substituiertes, gerad- oder verzweigtkettiges C₁₋₈-Alkyl ist.

6. Verbindung nach einem der vorangehenden Ansprüche, wobei X C=O ist.

7. Verbindung nach einem der Ansprüche 1 und 4-6, wobei die Verbindung Formel I besitzt und R₄ Amino ist.

8. Verbindung nach Anspruch 1 oder 2, die:
2-Amino-4-furan-2-yl-indeno[1,2-d]pyrimidin-5-on;
2-Amino-4-phenyl-indeno[1,2-d]pyrimidin-5-on;
2-Amino-4-thiophen-2-yl-indeno[1,2-d]pyrimidin-5-on;
2-Amino-4-(5-methylfuran-2-yl)-indeno[1,2-d]pyrimidin-5-on;
2,6-Diamino-4-furan-2-yl-indeno[1,2-d]pyrimidin-5-on;
9H-Indeno[2,1-c]pyridin-4-carbonitril, 3-Amino-1-furan-2-yl-9-oxo-;
9H-Indeno[2,1-c]pyridin-4-carbonsäure, 3-Amino-1-furan-2-yl-9-oxo-, 2-Dimethylaminoethylester;
9H-Indeno[2,1-c]pyridin-4-carbonsäure, 3-Amino-1-phenyl-9-oxo-, 2-Dimethylaminoethylester;
9H-Indeno[2,1-c]pyridin-4-carbonsäure, 3-Amino-1-furan-2-yl-9-oxo-, (2-Dimethylamino-1-methylethyl)-amid;
9H-Indeno[2,1-c]pyridin-4-carbonsäure, 3-Amino-1-furan-2-yl-9-oxo-, (2-Dimethylaminoethyl)-methylamid; oder
9H-Indeno[2,1-c]pyridin-4-carbonsäure, 3-Amino-1-furan-2-yl-9-oxo-, 1-Methylpyrrolidin-2-ylmethylester ist.

9. Pharmazeutische Zusammensetzung, die die Verbindung nach einem der Ansprüche 1 bis 8 und einen pharmazeutisch verträglichen Trägerstoff umfasst, wobei die Maßgabe von Anspruch 1 nicht zutrifft.

10. Verbindung nach einem der Ansprüche 1 bis 8 zur Verwendung in der Medizin, wobei die Maßgabe von Anspruch 1 nicht zutrifft.

11. Verbindung nach einem der Ansprüche 1 bis 8 oder pharmazeutische Zusammensetzung nach Anspruch 9 zur Verwendung bei der Behandlung eines Patienten mit, oder Verhinderung in einem Patienten von, einer Störung, die durch das Antagonisieren von Adenosin-A2a-Rezeptoren in geeigneten Zellen gelindert wird, wie etwa eine neurodegenerative Störung oder eine Bewegungsstörung, zum Beispiel Parkinson-Krankheit, Huntington-Krankheit, multiple Systematrophie, corticobasale Degeneration, Alzheimer-Krankheit und Altersdemenz, wobei die Maßgabe von Anspruch 1 nicht zutrifft.

## Revendications

1. Composé ayant la structure de la Formule I 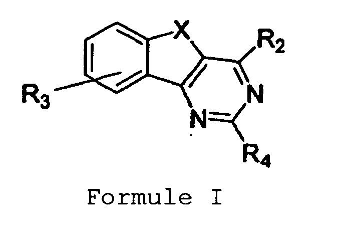 ou un sel pharmaceutiquement acceptable de celui-ci, où
(b) R₂ est sélectionné dans le groupe consistant en aryle optionnellement substitué, et en hétéroaryle optionnellement substitué;
(c) R₃ est de un à quatre groupes indépendamment sélectionné dans le groupe consistant en:
hydrogène, halo, alkyleC₁₋₈ à chaîne droite ou branchée, arylalkyle, cycloalkyleC₃₋₇, alcoxyC₁₋₈, cyano, carboalcoxy C₁₋₄, trifluorométhyle, alklylsulfonyleC₁₋₈, halogène, nitro, hydroxy, trifluorométhoxy, carboxylate C₁₋₈, aryle, hétéroaryle et hétérocyclyle, -NR₁₁R₁₂,
où R₁₁ et R₁₂ sont indépendamment sélectionnés parmi H, alkyle C₁₋₈ à chaîne droite ou branchée, arylalkyle, cycloalkyleC₃₋₇, carboxyalkyle, aryle, hétéroaryle et hétérocyclyle ou R₁₀ et R₁₁ pris ensemble avec l'azote forment un groupe hétéroaryle ou hétérocyclyle,
-NR₁₃COR₁₄,
où R₁₃ est sélectionné parmi hydrogène ou alkyle, et R₁₄ est sélectionné parmi hydrogène, alkyle, alkyle substitué, alcoxyC₁₋₃, carboxyalkyle, aryle, arylalkyle, hétéroaryle, hétérocyclyle, R₁₅R₁₆N(CH₂)ₚ-, ou R₁₅R₁₆NCO(CH₂)ₚ-, où R₁₅ et R₁₆ sont indépendamment sélectionnés parmi H, OH, alkyle et alcoxy, et p est un entier de 1 à 6,
dans lequel le groupe alkyle peut être substitué par carboxyle, alkyle, aryle, aryle substitué, hétérocyclyle, hétérocyclyle substitué, hétéroaryle, hétéroaryle substitué, acide hydroxamique, sulfonamide, sulfonyle, hydroxy, thiol, alcoxy ou arylalkyle, ou bien R₁₃ et R₁₄ pris ensemble avec le carbonyle forment un carbonyle contenant un groupe hétérocyclyle;
(d) R₄ est sélectionné dans le groupe consistant en hydrogène, alkyle C₁₋₆ à chaîne droite ou branchée, benzyle,
où les groupes alkyle et benzyle sont optionnellement substitués par un ou plusieurs groupes sélectionnés parmi cycloalkyleC₃₋₇, alcoxyC₁₋₈, cyano, carboalcoxy C₁₋₄, trifluorométhyle, alkylsulfonyle C₁₋₈, halogène, nitro, hydroxy, trifluorométhoxy, carboxylateC₁₋₈, amino, NR₁₇R₁₈, aryle et hétéroaryle,
-OR₁₇ et -NR₁₇R₁₈,
où R₁₇ et R₁₈ sont indépendamment sélectionnés parmi hydrogène, et alkyle C₁₋₆ ou aryle optionnellement substitué; et
(e) X est sélectionné parmi C=S, C=O; CH₂, CHOH, CHOR₁₉ ou CHNR₂₀R₂₁ où R₁₉, R₂₀ et R₂₁ sont sélectionnés parmi alkyle C₁₋₈ à chaîne droite ou branchée optionnellement substitué, où les substituants sur le groupe alkyle sont sélectionnés parmi alcoxyC₁₋₈, hydroxy, halogène, amino, cyano ou NR₂₂R₂₃ où R₂₂ et R₂₃ sont indépendamment sélectionnés dans le groupe consistant en hydrogène, alkyleC₁₋₈ à chaîne droite ou branchée, cycloalkyle C₃₋₇, benzyle, aryle, hétéroaryle ou NR₂₂R₂₃ pris ensemble forment un hétérocycle ou hétéroaryle;
dans lequel, à moins que cela ne soit indiqué autrement,
"alkyle" signifie un alkyle à chaîne droite, cyclique ou branchée contenant 1-20 atomes de carbone et optionnellement substitué par un ou plusieurs groupes comme halogène, OH, CN, mercapto, nitro, amino, alkyleC₁-C₈, alcoxyC₁-C₈, alkylthioC₁-C₈, alkyl-amino-C₁-C₈, di(alkyleC₁-C₈)amino, (mono-, di-, tri- et per-)halo-alkyle, formyle, carboxy, alcoxycarbonyle, alkyl-C₁-C₈-CO-O-, alkyl-C₁-C₈-CO-NH-, carboxamide, acide hydroxamique, sulfonamide, sulfonyle, thiole, aryle, arylalkyle(C₁-C₈), hétérocyclyle et hétéroaryle;
"aryle" ou "Ar", soit utilisé seul soit en tant que partie d'un groupe substituant, est un radical carbocyclique aromatique qui peut être substitué par le remplacement indépendant de 1 à 5 atomes de carbone sur celui-ci par halogène, OH, CN, mercapto, nitro, amino, alkyleC₁-C₈, alcoxyC₁-C₈, alkylthioC₁-C₈, alkyl-amino-C₁-C₈, di(alkyleC₁-C₈)amino, (mono-, di-, tri- et per-) halo-alkyle, formyle, carboxy, alcoxycarbonyle,alkyl-C₁-C₈-CO-O-, alkyl-C₁-C₈-CO-NH-, ou carboxamide; et
"hétérocycle", "hétérocyclique" et "hétérocyclo" se réfèrent à un groupe cyclique optionnellement substitué, entièrement ou partiellement saturé qui est, par exemple, un système de cycle monocyclique de 4 à 7 membres, bicyclique de 7 à 11 membres, ou tricyclique de 10 à 15 membres, qui possède au moins un hétéroatome dans au moins un atome de carbone contenant le cycle, et où chaque cycle du groupe hétérocyclique contenant un hétéroatome peut avoir 1, 2 ou 3 hétéroatomes sélectionnés parmi les atomes d'azote, atomes d'oxygène et atomes de soufre, où les hétéroatomes d'azote et de soufre peuvent également être oxydés optionnellement, et les atomes d'azote peuvent optionnellement être quaternisés;
à condition que ledit composé ne soit pas: 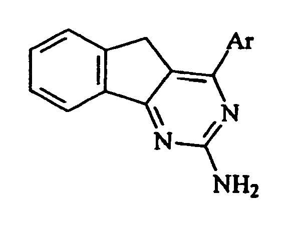 où Ar est phényle, *p*-méthoxyphényle, *m*-méthoxyphényle, *p*-bromophényle, *m*-bromophényle, *p*-chlorophényle, *o-*chlorophényle, 1-naphtyle, 2-naphtyle, 2-thiényle ou 2-furanyle; ou bien 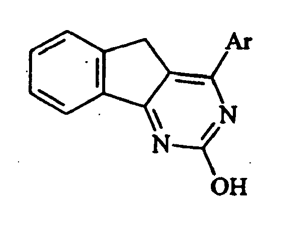 où Ar est *p*-chlorophényle, 1-naphtyle ou 2-thiényle.

2. Composé ayant la structure de la Formule II 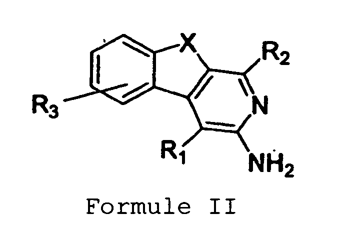 ou un sel pharmaceutiquement acceptable de celui-ci, dans lequel
(a) R₁ est sélectionné dans le groupe consistant en
(i) -COR₅, où R₅ est sélectionné parmi H, alkyle C₁₋₈ à chaîne droite ou branchée optionnellement substitué, aryle optionnellement substitué et arylalkyle optionnellement substitué;
où les substituants sur le groupe alkyle, aryle et arylalkyle sont sélectionnés parmi alcoxyC₁₋₈, phénylacétyloxy, hydroxy, halogène, p-tosyloxy, mésyloxy, amino, cyano, carboalcoxy ou NR₇R₈, où R₇ et R₈ sont indépendamment sélectionnés dans le groupe consistant en hydrogène, alkyleC₁-₈ à chaîne droite ou branchée, cycloalkyle C₃₋₇, benzyle, aryle ou hétéroaryle ou NR₇R₈ pris ensemble forment un hétérocycle ou hétéroaryle;
(ii) COOR₅, où R₅ est comme défini ci-dessus;
(iii)cyano;
(iv) -CONR₉R₁₀ où R₉ et R₁₀ sont indépendamment sélectionnés parmi H, alkyle C₁₋₈ de chaîne droite ou branchée, cycloalkyle C₃₋₇, trifluorométhyle, hydroxy, alcoxy, acyle, alkylcarbonyle, carboxyle, arylalkyle, aryle, hétéroaryle et hétérocyclyle; dans lequel les groupes alkyle, cycloalkyle, alcoxy, acyle, alkylcarbonyle, carboxyle, arylalkyle, aryle, hétéroaryle et hétérocyclyle peuvent être substitués par carboxyle, alkyle, aryle, aryle substitué, hétérocyclyle, hétérocyclyle substitué, hétéroaryle, hétéroaryle substitué, acide hydroxamique, sulfonamide, sulfonyle, hydroxy, thiol, amino, alcoxy ou arylalkyle, ou bien R₉ et R₁₀ pris ensemble avec l'azote auquel ils sont fixés forment un groupe hétérocycle ou hétéroaryle;
(v) alkyleC₁₋₈ à chaîne droite ou branchée optionnellement substitué;
dans lequel des substituants sur le groupe alkyle sont sélectionnés parmi alcoxy C₁₋₈, phénylacétyloxy, hydroxy, halogène, p-tosyloxy, mésyloxy, amino, cyano, carboalcoxy, carboxyle, aryle, hétérocyclyle, hétéroaryle, sulfonyle, thiol, alkylthio ou NR₇R₈, où R₇ et R₈ sont comme définis ci-dessus;
(b) R₂ est sélectionné dans le groupe consistant en alkyle optionnellement substitué, aryle optionnellement substitué, hétéroaryle optionnellement substitué, hétérocyclyle optionnellement substitué et cycloalkyle C₃₋₇ optionnellement substitué, alcoxy C₁₋₈, aryloxy, alkylsulfonyleC₁₋₈, arylsulfonyle, arylthio, alkylthioC₁₋₈ ou -NR₂₄R₂₅
où R₂₄ et R₂₅ sont indépendamment sélectionnés parmi H, alkyle C₁₋₈ à chaîne droite ou branchée, arylalkyle, cycloalkyle C₃₋₇, carboxylalkyle, aryle, hétéroaryle et hétérocyclyle ou bien R₂₄ et R₂₅ pris ensemble avec l'azote forment un groupe hétéroaryle ou hétérocyclyle,
(c) R₃ est de un à quatre groupes indépendamment sélectionnés dans le groupe consistant en:
hydrogène, halo, alkyle C₁₋₈ à chaîne droite ou branchée, arylalkyle, cycloalkyleC₃₋₇, alcoxyC₁₋₈, cyano, carboalCOxyC₁₋₄, trifluorométhyle, alkylsulfonyleC₁₋₈, halogène, nitro, hydroxy, trifluorométhoxy, carboxylate C₁₋₈, aryle, hétéroaryle et hétérocyclyle, -NR₁₁R₁₂,
où R₁₁ et R₁₂ sont indépendamment sélectionnés parmi H, alkyle C₁₋₈ à chaîne droite ou branchée, arylalkyle, cycloalkyleC₃₋₇, carboxyalkyle, aryle, hétéroaryle et hétérocyclyle, ou bien R₁₀ et R₁₁ pris ensemble avec l'azote forment un groupe hétéroaryle ou hétérocyclyle,
-NR₁₃COR₁₄,
où R₁₃ est sélectionné parmi hydrogène ou alkyle et R₁₄ est sélectionné parmi hydrogène, alkyle, alkyle substitué, alcoxyC₁₋₃, carboxyalkyle, aryle, arylalkyle, hétéroaryle, hétérocyclyle, R₁₅R₁₆N (CH₂)ₚ-, ou R₁₅R₁₆NCO(CH₂)ₚ-, où R₁₅ et R₁₆ sont indépendamment sélectionnés parmi H, OH, alkyle et alcoxy, et p est un entier de 1-6,
où le groupe alkyle peut être substitué par carboxyle, alkyle, aryle, aryle substitué, hétérocyclyle, hétérocyclyle substitué, hétéroaryle, hétéroaryle substitué, acide hydroxamique, sulfonamide, sulfonyle, hydroxy, thiol, alcoxy ou arylalkyle, ou bien R₁₃ et R₁₄ pris ensemble avec le carbonyle forment un groupe hétérocyclyle contenant du carbonyle;
(e) X est sélectionné parmi C=S, C=O; CH₂, CHOH, CHOR₁₉ ou CHNR₂₀R₂₁, où R₁₉, R₂₀ et R₂₁ sont sélectionnés parmi alkyleC₁₋₈ à chaîne droite ou ramifiée optionnellement substitué, où les substituants sur le groupe alkyle sont sélectionnés parmi alcoxyC₁₋₈, hydroxy, halogène, amino, cyano ou NR₂₂R₂₃, où R₂₂ et R₂₃ sont indépendamment sélectionnés dans le groupe consistant en hydrogène, alkyleC₁₋₈ à chaîne droite ou branchée, cycloalkyleC₃₋₇, benzyle, aryle, hétéroaryle, ou NR₂₂R₂₃ pris ensemble forment un hétérocycle ou hétéroaryle;
dans lequel, à moins que cela ne soit indiqué autrement,
"alkyle" signifie un alkyle à chaîne droite, cyclique ou branchée contenant 1-20 atomes de carbone et optionnellement substitué par un ou plusieurs groupes comme halogène, OH, CN, mercapto, nitro, amino, alkyleC₁-C₈, alcoxyC₁-C₈, alkylthioC₁-C₈, alkyl-amino-C₁-C₈, di(alkyleC₁-C₈)amino, (mono-, di-, tri- et per-) halo-alkyle, formyle, carboxy, alcoxycarbonyle, alkyl-C₁-C₈-CO-O-, alkyl-C₁-C₈-CO-NH-, carboxamide, acide hydroxamique, sulfonamide, sulfonyle, thiole, aryle, arylalkyle(C₁-C₈), hétérocyclyle et hétéroaryle;
"aryle" ou "Ar", soit utilisé seul soit en tant que partie d'un groupe substituant, est un radical carbocyclique aromatique qui peut être substitué par le remplacement indépendant de 1 à 5 atomes de carbone sur celui-ci par halogène, OH, CN, mercapto, nitro, amino, alkyleC₁-C₈, alcoxyC₁-C₈, alkylthioC₁-C₈, alkyl-amino-C₁-C₈, di(alkyleC₁-C₈) amino, (mono-, di-, tri- et per-) halo-alkyle, formyle, carboxy, alcoxycarbonyle,alkyl-C₁-C₈-CO-O-, alkyl-C₁-C₈-CO-NH-, ou carboxamide; et
"hétérocycle", "hétérocyclique" et "hétérocyclo" se réfèrent à un groupe cyclique optionnellement substitué, entièrement ou partiellement saturé qui est, par exemple, un système de cycle monocyclique de 4 à 7 membres, bicyclique de 7 à 11 membres, ou tricyclique de 10 à 15 membres, qui possède au moins un hétéroatome dans au moins un atome de carbone contenant le cycle, et où chaque cycle du groupe hétérocyclique contenant un hétéroatome peut avoir 1, 2 ou 3 hétéroatomes sélectionnés parmi les atomes d'azote, atomes d'oxygène et atomes de soufre, où les hétéroatomes d'azote et de soufre peuvent également être oxydés optionnellement, et les atomes d'azote peuvent optionnellement être quaternisés;
à condition que lorsque R₁ est un cyano, R₂ ne soit pas phényle.

3. Composé selon la revendication 2, où R₂ est hétéroaryle optionnellement substitué ou aryle optionnellement substitué.

4. Composé selon la revendication 1 ou la revendication 3, où R₂ est un furanne optionnellement substitué.

5. Composé selon l'une quelconque des revendications précédentes, où R₁ est COOR₅ et R₅ est un alkyle C₁₋₈ à chaîne droite ou branchée optionnellement substitué.

6. Composé selon l'une quelconque des revendications précédentes, où X est C=O.

7. Composé selon l'une quelconque des revendications 1 et 4 à 6, où le composé est de la Formule I, et R₄ est amino.

8. Composé selon la revendication 1 ou 2, qui est:
2-amino-4-furanne-2-yl-indéno[1,2-d]pyrimidin-5-one;
2-amino-4-phényl-indéno[1,2-d]pyrimidin-5-one;
2-amino-4-thiophen-2-yl-indéno[1,2-d]pyrimidin-5-one;
2-amino-4-(5-méthyl-furanne-2-yl)-indéno[1,2-d]pyrimidin-5-one;
2,6-diamino-4-furanne-2-yl-indéno[1,2-d]pyrimidin-5-one;
9*H*-indéno[2,1-c]pyridine-4-carbonitrile, 3-amino-1-furanne-2-yl-9-oxo;
3-amino-1-furanne-2-yl-9-oxo-, 2-diméthylamino-éthyl ester de l'acide 9*H*-indéno[2,1-c]pyridine-4-carboxylique;
3-amino-1-furanne-2-yl-9-oxo-, 2-diméthylamino-éthyl ester de l'acide 9H-indéno[2,1-c]pyridine-4-carboxylique;
3-amino-furanne-2-yl-9-oxo, (2-diméthylamino-1-méthyl-éthyl)-amide de l'acide 9*H*-indéno[2,1-c]pyridine-4-carboxylique;
3-amino-1-furanne-2-yl-9-oxo-, (2-diméthylamino-éthyl)-méthyl-amide de l'acide 9*H*-indéno[2,1-c]pyridine-4-carboxylique;
3-amino-1-furanne-2-yl-9-oxo-, 1-méthyl-pyrrolidin-2-ylméthyl ester de l'acide 9*H*-indéno[2,1-c]pyridine-4-carboxylique.

9. Composition pharmaceutique comprenant le composé selon l'une quelconque des revendications 1 à 8 et un support pharmaceutiquement acceptable, dans laquelle la condition de la revendication 1 ne s'applique pas.

10. Composé selon l'une quelconque des revendications 1 à 8 pour utilisation en médecine, où la condition de la revendication 1 ne s'applique pas.

11. Composé selon l'une quelconque des revendications 1 à 8 ou une composition pharmaceutique de la revendication 8 utilisée pour le traitement d'un sujet ayant, ou prévenant dans un sujet, un trouble amélioré par des récepteurs antagonisants de l'Adénosine A2a dans des cellules appropriées, comme une maladie neurodégénérative ou un trouble de mouvement, par exemple la maladie de Parkinson, chorée de Huntington, maladie de Shy-Drager, dégénération cortico-basale, maladie d'Alzheimer et démence sénile, où la condition de la revendication 1 ne s'applique pas.
